# EUROPEAN PATENT SPECIFICATION

(11) **EP 2 523 972 B1**
(45) Date of publication and mention of the grant of the patent: **30.03.2016**
(21) Application number: 11700101.6
(22) Date of filing: 12.01.2011
(51) Int. Cl.: C07K 16/24, C07K 16/28, C07K 16/46, C07K 16/18

(54) **THREEVALENT ANTIBODIES**
TRIVALENTE ANTIKÖRPER
ANTICORPS TRIVALENTS

(30) Priority: 12.01.2010 GB 201000467
(43) Date of publication of application: 21.11.2012
(73) Proprietor: UCB Biopharma SPRL, 1070 Brussels (BE)
(72) Inventor: ADAMS, Ralph, Slough, Berkshire SL1 3WE (GB); DAVE, Emma, Slough, Berkshire SL1 3WE (GB)
(74) Representative: Blanchard, Amanda Jane
(86) International application number: PCT/EP2011/050333
(87) International publication number: WO 2011/086091

(56) References cited:
- EP-A1- 2 050 764
- WO-A1-2009/018386
- WO-A1-2009/040562
- WO-A1-2009/068628
- WO-A1-2010/136172
- WO-A1-2011/030107
- WO-A2-2009/021754
- US-A1- 2007 071 675

## Description

The present disclosure relates to antibodies with three antigen binding sites, pharmaceutical compositions comprising the same, use of the each of the same in therapy and methods for preparing said antibodies.

Multivalent antibodies are known (e.g. WO 2009/018386, EP 2050764, US 2007/071675, WO 2009/040562, WO 2009/02154, WO 2009/068628). However, even though the basic concept was disclosed a number of years ago, there have been practical difficulties associated with exploiting the technology and thus it has not been widely adopted for the preparation of pharmaceutical biologic products in development.

A non-natural/non-native antibody format can be difficult to express, which may significantly increase the cost of goods to an untenable level. The formats may increase the immunogenicity or reduce the *in vivo* stability in comparison to a standard antibody or fragment and/or may have undesirable pharmokinetics.

In particular the problems associated with preparing homogenous products have been a concern for non-natural formats. If, for example, there is more than one permutation for combining the component monomers then mixtures can result. Thus elaborate purification methods may be required to isolate the desired/target entity at satisfactory purity levels.

This has been addressed in a number of ways, for example using short linkers in the production of bispecific diabodies was said to aid appropriate dimerisation. However, data has shown that the orientation of the variable domains can influence expression of the format and the formation of active binding sites.

One approach to force the assembly in the desired arrangement or orientation is referred to as the "knob-in-hole" method, in which a large "knob" is introduced in the VH domain by, for example in some antibodies exchanging valine 137 with the large residue phenylalanine and replacing leucine 45 with tryptophan. A complementary hole can be introduced, for example in the VL domain by, in some antibodies, mutating phenylalanine 98 to methionine and tryptophan 87 to alanine. However, reduced antigen-binding activity was observed for several constructs.

The present disclosure provides an entity wherein inappropriate pairing of the components may be minimised.

Thus there is provided a recombinant antibody or a heavy/light chain component thereof comprising:
a heavy chain comprising a C_{H1} constant region fragment located between a first and
second variable domain, and a third variable domain linked directly or indirectly to the first or second variable domain,
with the proviso that the heavy chain only contains one C_{H1} and only contains three variable domains, and
a light chain comprising at least a C_{L} domain located between a first and second variable domain, and a third variable domain linked directly or indirectly to the first or second variable domain,
with the proviso that the light chain only contains one C_{L} domain and only contains three variable domains, and
wherein said heavy and light chains are aligned to provide a first binding site formed by the first variable domains in the light and heavy chain, a second binding site formed by the second variable domains in the light and heavy chain, and a third binding site formed by the third variable domains in the light and heavy chain.

In one embodiment at least two of the said three binding sites are specific for different epitopes.

In one embodiment there is provided a recombinant antibody or a heavy/light chain component thereof comprising:
a heavy chain comprising a C_{H1} constant region fragment located between a first and
second variable domain which are not a cognate pair, and a third variable domain which is not a cognate pair with the first and second variable domains, wherein said third variable domain is linked directly or indirectly to the first or second variable domain,
with the proviso that the heavy chain only contains one C_{H1} and only contains three variable domains, and
a light chain comprising at least a C_{L} domain located between a first and second variable domain which are not a cognate pair, and a third variable domain which is not a cognate pair with the first and second variable domains, wherein said third variable domain is linked directly or indirectly to the first or second variable domain,
with the proviso that the light chain only contains one C_{L} domain and only contains three variable domains, and
wherein said heavy and light chains are aligned to provide a first binding site formed by the first variable domains in the light and heavy chain, a second binding site formed by the second variable domains in the light and heavy chain, and a third binding site formed by the third variable domains in the light and heavy chain.

Preferably each variable domain in the heavy chain is a VH domain and each variable domain in the light chain is a VL domain.

The heavy chain construct of the present disclosure may, for example be represented as follows:

V_{H1}C_{H1}L₁V_{H2}L₂V_{H3} (Ia)

or

V_{H3}L₂V_{H1}C_{H1}L₁V_{H2} (Ib)

wherein:
V_{H1} is a first variable domain;
C_{H1} is a C_{H1} constant region domain;
L₁ is a linker or a bond;
V_{H2} is a second variable domain;
L₂ is a linker or a bond, and
V_{H3} is a third variable domain, and
the light chain construct of the present disclosure may be represented as:

V_{L1}C_{L}L₁V_{L2}L₂V_{L3} (IIa)

or

V_{L3}L_{L}V_{L1}C_{L}L₁V_{L2} (IIb)

V_{L1} is a first variable domain;
C_{L} is a C_{L} constant region domain;
L₁ is a linker or a bond;
V_{L2} is a second variable domain;
L₂ is a linker or a bond; and
V_{L3} is a third variable domain; and
the heavy and light chain pair such that V_{H1} and V_{L1} form a binding domain, V_{H2} and V_{L2} form a binding domain and V_{H3} and V_{L3} form a binding domain.

In one embodiment the present invention provides a recombinant antibody or heavy/light chain component thereof comprising or consisting of:
a heavy chain represented as:

   V_{H1}C_{H1}L₁V_{H2}L₂V_{H3}

   wherein:
V_{H1} is a first variable domain;
C_{H1} is a C_{H1}constant region domain;
L₁ is a linker or a bond;
V_{H2} is a second variable domain;
L₂ is a linker or a bond, and
V_{H3} is a third variable domain, and
a light chain represented as:

V_{L1}C_{L}L₁V_{L2}L₂V_{L3}

wherein:
V_{L1} is a first variable domain;
C_{L} is a C_{L}constant region domain;
L₁ is a linker or a bond;
V_{L2} is a second variable domain;
L₂ is a linker or a bond; and
V_{L3} is a third variable domain; and
the heavy and light chain pair such that V_{H1} and V_{L1} form a binding domain, V_{H2} and V_{L2} form a binding domain and V_{H3} and V_{L3} form a binding domain.

Advantageously, the provision of only one C_{H1} in the heavy chain and only one C_{L} in the light chain facilitates appropriate pairing.

### Brief Description of the Figures

Figures 1-6 show antibody or heavy/light chain component sequences according to the present invention.
Figure 7 shows SDS-PAGE analysis of FabA-(3xG4S)-645dsFv-(3xG4S)-652dsFv) under (a) reducing conditions and (b) non-reducing conditions.

In one embodiment the heavy chain is format (Ia) and the light chain is (IIa) as shown above.

Antibody as employed herein is intended to refer to the format comprising two heavy chains and two light chains.

A heavy/light chain component according to the present disclosure is a heavy chain and associated light chain.
The heavy chain as employed herein is the chain which comprises the C_{H1} region.
The light chain as employed herein comprises the C_{L} region.
The antibody or fragment does not comprise an Fc region.
The heavy/light chain component does not comprise an Fc region.

The variable domains are provided in each chain such that they form pre-defmed pairs with suitable/adequate binding to a target antigen i.e. each pair form a binding site or domain. Suitable variable domain pairs may be identified by any means possible, for example including generation of antibodies in hosts and screening of B cells. Alternatively suitable pairs may be identified by phage display. In one embodiment the variable domain pair has affinity for a target antigen of 100nM or less, such as 50nM or less, in particular 1 nM or less.

Phage display methods known in the art and include those disclosed by Brinkman et al., J. Immunol. Methods, 1995, 182, 41-50; Ames et al., J. Immunol. Methods, 1995, 184, 177-186; Kettleborough et al. Eur. J. Immunol., 1994, 24, 952-958; Persic et al., Gene, 1997 187, 9-18; and Burton et al., Advances in Immunology, 1994, 57, 191-280; WO 90/02809; WO 91/10737; WO 92/01047; WO 92/18619; WO 93/11236; WO 95/15982; and WO 95/20401; and US 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743; and 5,969,108.

Transgenic mice, or other organisms, including other mammals, may be used to generate humanized antibodies.

In one embodiment the variable domain pair which form a binding site is a cognate pair. Cognate pair as employed herein is intended to refer to a natural pair of variable domains, that is to say isolated from a single antibody or antibody expressing cell, such as a B cell.

In one example the cognate pair are a complementary VH/VL pair which bind the antigen co-operatively i.e. they are a complementary VH/VL pair.
Typically the cognate pair will be a VH/VL pair derived from the same antibody.
In one example the cognate pair are a pair of variable domains isolated as a pair from a 'library of pairs', such as a Fab phage display library.
In one example the VH/VL pair are monospecific.

Variable domains may have been optimized and/or humanized.

Optimised/humanized variable domains derived from a cognate pair will still be considered a cognate pair after optimization/humanization.

C_{L} as employed herein refers to the constant region portion in the light chain, which may be a naturally occurring light chain constant region.

Fused as employed herein is intended to refer to a continuous amino acid sequence that is uninterrupted, i.e. linked directly via a peptide bond, for example directly to the sequence of the variable domain or conversely the constant region fragment and not joined by a linker. Inserting a non-natural peptide linker into an amino acid sequence disrupts the sequence and thus a peptide linker containing sequence would not be considered to fuse the relevant portions together within the meaning of the present specification. The addition a natural peptide linker would also be considered interruption of the amino acid sequence, if it cannot be considered to form part of the sequence of one or more of the relevant components, such as a variable domain or constant region fragment.

In one embodiment the antibody or heavy/light chain component thereof avidly binds the target antigen.

The antibody or heavy/light chain component thereof of the present invention comprises or consists of 3 binding sites each made up of a VHNL pair.

In one embodiment the antibody or heavy/light chain component thereof according to the present disclosure is mono-specific. Monospecific as employed herein is intended to refer to the fact that all the binding sites bind the same target antigen. In one aspect of this embodiment all the binding sites bind the same epitope(s) of said antigen, for example, each VH/VL pair is the same. In an alternative embodiment at least two binding sites bind different epitopes on the target antigen.

In one embodiment an antibody or heavy/light chain component according to the present disclosure is bispecific such that at least two binding sites specifically bind different or distinct antigens.

In one embodiment each binding site specifically binds different or distinct antigens i.e. the antibody or heavy/light chain component is trispecific.

Specifically binds as employed herein is intended to refer to an antibody having high affinity for a target antigen (to which it is specific) and which binds antigens to which it is not specific with a low or much lower affinity (or not at all). Methods of measuring affinity are known to those skilled in the art and include such assays as BIAcore.

Different epitopes are distinct epitopes, but may, for example be on the same antigen. In one or more embodiments the different epitopes are on different antigens.

In one embodiment each constant region fragment is also linked via a peptide, for example an artificial/non-naturally occurring linker such as sequence in Table 2, to a variable domain, for example which is a non-cognate pair to the variable domain fused thereto. Thus, for example C_{H1} is linked indirectly, such as via a peptide to V_{H2} and C_{L} is linked indirectly, such as via a peptide to V_{L2}.

In one embodiment the third variable domain in each chain is linked indirectly to the second variable domain in that chain, for example by a peptide, such that V_{H2} is linked to VH₃ by a peptide and V_{L2} is linked to V_{L3} by a peptide.

In one embodiment the variable domains which form a binding site, (for example of a cognate pair) are not linked by a disulfide bond. In one embodiment there are no disulfide bonds between the variable domains of any variable domains pairs which form binding sites (for example no disulfide bonds between cognate pairs).

In one embodiment the variable domains of at least one variable domain pair such as a cognate pair are linked by a disulfide bond, for example a disulfide bond is present between V_{H2} and V_{L2}. Alternatively or additionally a disulfide bond is present between V_{H3} and V_{L3}. Alternatively or additionally to each of the aforementioned a disulfide bond is present between V_{H1} and V_{L1}. The presence of at least one disulfide bond between a variable domain pair may minimise aggregation and further optimize the stability of the constructs prepared.

In one embodiment the disulfide bond is between (unless the context indicates otherwise Kabat numbering is employed in the list below, wherever reference is made to Kabat numbering the relevant reference is Kabat et al., 1987, in Sequences of Proteins of Immunological Interest, US Department of Health and Human Services, NIH, USA):
- VH37 + VL95C see for example Protein Science 6, 781-788 Zhu et al (1997);
- VH44 + VL100 see for example; Biochemistry 33 5451-5459 Reiter et al (1994); or Journal of Biological Chemistry Vol. 269 No. 28 pp.18327-18331 Reiter et al (1994); or Protein Engineering, vol.10 no.12 pp.1453-1459 Rajagopal et al (1997);
- VH44 + VL105 see for example J Biochem. 118, 825-831 Luo et al (1995);
- VH45 + VL87 see for example Protein Science 6, 781-788 Zhu et al (1997);
- VH55 + VL101 see for example FEBS Letters 377 135-139 Young et al (1995);
- VH100 + VL50 see for example Biochemistry 29 1362-1367 Glockshuber et al (1990);
- VH100b + VL49;
- VH98 + VL 46 see for example Protein Science 6, 781-788 Zhu et al (1997);
- VH101 + VL46
- VH105 + VL43 see for example; Proc. Natl. Acad. Sci. USA Vol. 90 pp.7538-7542 Brinkmann et al (1993); or Proteins 19, 35-47 Jung et al (1994) or
- VH106 + VL57 see for example FEBS Letters 377 135-139 Young et al (1995).
The amino acid pairs listed above are in the positions conducive to replacement by cysteines such that disulfide bonds can be formed. Cysteines can be engineered into these positions by known techniques. Introduction of engineered cysteines can be performed using any method known in the art. These methods include, but are not limited to, PCR extension overlap mutagenesis, site-directed mutagenesis or cassette mutagenesis (see, generally, Sambrook et al., Molecular Cloning, A Laboratory Manual, Cold Spring Harbour Laboratory Press, Cold Spring Harbour, NY, 1989; Ausbel et al., Current Protocols in Molecular Biology, Greene Publishing & Wiley-Interscience, NY, 1993). Site-directed mutagenesis kits are commercially available, e.g. QuikChange® Site-Directed Mutagenesis kit (Stratagen, La Jolla, CA). Cassette mutagenesis can be performed based on Wells et al., 1985, Gene, 34:315-323. Alternatively, mutants can be made by total gene synthesis by annealing, ligation and PCR amplification and cloning of overlapping oligonucleotides.

Accordingly in one embodiment a variable domain pair (VH/VL) of the present invention may be linked by a disulfide bond between two cysteine residues, one in VH and one in VL, wherein the position of the pair of cysteine residues is selected from the group consisting of VH37 and VL95, VH44 and VL100, VH44 and VL105, VH45 and VL87, VH100 and VL50, VH100b and VL49, VH98 and VL46, VH101 and VL46, VH105 and VL43 and VH106 and VL57.

In one embodiment a variable domain pair (VH/VL) of the present invention may be linked by a disulfide bond between two cysteine residues, one in VH and one in VL, which are outside of the CDRs wherein the position of the pair of cysteine residues is selected from the group consisting of VH37 and VL95, VH44 and VL100, VH44 and VL105, VH45 and VL87, VH100 and VL50, VH98 and VL46, VH105 and VL43 and VH106 and VL57.

In one embodiment a variable domain pair (VH/VL) of the present invention may be linked by a disulfide bond between two cysteine residues, one in VH and one in VL, which are outside of the CDRs wherein the position of the pair of cysteine residues is selected from the group consisting of VH37 and VL95, VH44 and VL105, VH45 and VL87, VH100 and VL50, VH98 and VL46, VH105 and VL43 and VH106 and VL57.

In one embodiment a variable domain pair (VH/VL) of the present invention may be linked by a disulfide bond between two cysteine residues wherein the cysteine residue of VH is at position 44 and the cysteine residue of VL is at position 100.

Typically the cysteine pairs are engineered into those positions in VH and VL, accordingly in one embodiment a variable domain pair (VH/VL) of the present invention may be linked by a disulfide bond between two engineered cysteine residues, one in VH and one in VL, wherein the position of the pair of engineered cysteine residues is selected from the group consisting of VH37 and VL95, VH44 and VL100, VH44 and VL105, VH45 and VL87, VH100 and VL50, VH100b and VL49, VH98 and VL46, VH101 and VL46, VH105 and VL43 and VH106 and VL57.

In one embodiment a variable domain pair (VH/VL) of the present invention may be linked by a disulfide bond between two engineered cysteine residues, one in VH and one in VL, which are outside of the CDRs wherein the position of the pair of engineered cysteine residues is selected from the group consisting of VH37 and VL95, VH44 and VL100, VH44 and VL105, VH45 and VL87, VH100 and VL50, VH98 and VL46, VH105 and VL43 and VH106 and VL57.

In one embodiment the variable domain pair (VH/VL) is linked by a disulfide bond between two engineered cysteine residues, one in VH and one in VL, which are outside of the CDRs wherein the position of the pair of engineered cysteine residues is selected from the group consisting of VH37 and VL95, VH44 and VL105, VH45 and VL87, VH100 and VL50, VH98 and VL46, VH105 and VL43 and VH106 and VL57.

In one embodiment the variable domain pair (VH/VL) is linked by a disulfide bond between two engineered cysteine residues wherein the engineered cysteine residue of VH is at position 44 and the engineered cysteine residue of VL is at position 100.

In one embodiment a "natural" disulfide bond is present between C_{H1} and C_{L}. The C_{L} domain is derived from either Kappa or Lambda. The natural position for a bond forming 'interchain' cysteine in the latter is 214 in human cKappa and cLambda (Kabat numbering 4th edition 1987).

The exact location of the bond forming 'interchain' cysteine in C_{H1} depends on the particular domain actually employed. Thus, for example in human gamma-1 the natural position of the interchain cysteine forming the disulfide bond is located at position 233 (Kabat numbering 4th edition 1987). The position of the bond forming cysteine for other human isotypes such as gamma 2, 3, 4, IgM and IgD are known, for example 127.

In one embodiment the antibody or heavy/light chain component thereof according to the disclosure has a disulfide bond in a position equivalent to, or corresponding to that in the naturally occurring C_{H1} and C_{L}.

In one embodiment constant region comprising C_{H} or C_{L} has a disulfide bond which is in a non-naturally occurring position. This may be engineered into the molecule by introducing cysteine(s) into the amino acid chain at the positions required. This non-natural disulfide bond is in addition to or as an alternative to the natural disulfide bond present between C_{H} and C_{L}.

In one or more embodiments herein there are no interchain disulfide bonds in the C_{H1} and C_{L} regions.

Alternatively one or more embodiments herein may be provided with one or more (such as two) disulfide bonds between the C_{H1} and C_{L} regions, such as in the hinge region thereof.

In certain embodiments in each chain, each constant region fragment is fused to the first variable domain, for example C_{H1} is fused to V_{H1} and C_{L} is fused to V_{L1}. This in effect provides a Fab type arrangement. i.e. the C-terminus of V_{H1} is fused to the N-terminus of C_{H1} and the C-terminus of V_{L1} is fused to the N-terminus of C_{L}.

In one embodiment the constant region fragment, for example in the heavy chain, comprises a C_{H1} domain. In one embodiment the constant region fragment consists of a C_{H1} domain.

The C_{H}1 may be derived from human IgA, IgD, IgE, IgG (such as IgG1, IgG2, IgG3, IgG4) or IgM domains and isotypes thereof.

In one embodiment the constant region fragment consists of a C_{H1} domain having the sequence given in SEQ ID NO:66.

In one embodiment the light chain comprises a C_{L} domain. In one embodiment the constant region in the light chain consists of a C_{L} domain, which is either cKappa or cLambda.

In one embodiment the constant region fragment consists of a CL domain having the sequence given in SEQ ID NO:77.

In one embodiment the single chain components (a light and heavy chain pairing) are joined to each other to provide an antibody by a disulfide bond, for example, by incorporation of a hinge region in the heavy chain.

When a construct according to the present disclosure comprises a hinge, modified hinges may be employed as per Table 1.

A number of modified hinge regions have already been described for example, in US5,677,425, US6642356, WO9915549, WO2005003170, WO2005003169, WO2005003170, WO9825971 and WO2005003171 and these are incorporated herein by reference. The hinge will usually be located between the second variable domain in the heavy chain and C_{H1}. Particular examples of hinges include those shown in Table 1.

**Table 1. Hinge linker sequences**

| **SEQ ID NO:** | **SEQUENCE** |
|---|---|
| 1 | DKTHTCAA |
| 2 | DKTHTCPPCPA |
| 3 | DKTHTCPPCPATCPPCPA |
| 4 | DKTHTCPPCPATCPPCPATCPPCPA |
| 5 | DKTHTCPPCPAGKPTLYNSLVMSDTAGTCY |
| 6 | DKTHTCPPCPAGKPTHVNVSVVMAEVDGTCY |
| 7 | DKTHTCCVECPPCPA |
| 8 | DKTHTCPRCPEPKSCDTPPPCPRCPA |
| 9 | DKTHTCPSCPA |

The arrangement of C_{L} in the light chain and C_{H1} in the heavy chain is thought to minimize inappropriate dimerisation.

The inventors believe that by providing variable domains as cognate pairs in the final construct this optimises and maintains the antigen binding properties of the binding site formed by the relevant pair.

The disulfide bridges in the cognate pairs may be advantageous in that they assist in stabilizing the format.
Examples of suitable peptide linkers are given below, for example in Table 2.

**Table 2. Flexible linker sequences**

| **SEQ ID NO:** | **SEQUENCE** |
|---|---|
| 10 | SGGGGSE |
| 11 | DKTHTS |
| 12 | (S)GGGGS |
| 13 | (S)GGGGSGGGGS |
| 14 | (S)GGGGSGGGGSGGGGS |
| 15 | (S)GGGGSGGGGSGGGGSGGGGS |
| 16 | (S)GGGGSGGGGSGGGGSGGGGSGGGGS |
| 17 | AAAGSG-GASAS |
| 18 | AAAGSG-XGGGS-GASAS |
| 19 | AAAGSG-XGGGSXGGGS -GASAS |
| 20 | AAAGSG- XGGGSXGGGSXGGGS -GASAS |
| 21 | AAAGSG- XGGGSXGGGSXGGGSXGGGS-GASAS |
| 22 | AAAGSG-XS-GASAS |
| 23 | PGGNRGTTTTRRPATTTGSSPGPTQSHY |
| 24 | ATTTGSSPGPT |
| 25 | ATTTGS |
| | GS |
| 27 | EPSGPISTINSPPSKESHKSP |
| 28 | GTVAAPSVFIFPPSD |
| 29 | GGGGIAPSMVGGGGS |
| 30 | GGGGKVEGAGGGGGS |
| 31 | GGGGSMKSHDGGGGS |
| 32 | GGGGNLITIVGGGGS |
| 33 | GGGGVVPSLPGGGGS |
| 34 | GGEKSIPGGGGS |
| 35 | RPLSYRPPFPFGFPSVRP |
| 36 | YPRSIYIRRRHPSPSLTT |
| 37 | TPSHLSHILPSFGLPTFN |
| 38 | RPVSPFTFPRLSNSWLPA |
| 39 | SPAAHFPRSIPRPGPIRT |
| 40 | APGPSAPSHRSLPSRAFG |
| 41 | PRNSIHFLHPLLVAPLGA |
| 42 | MPSLSGVLQVRYLSPPDL |
| 43 | SPQYPSPLTLTLPPHPSL |
| 44 | NPSLNPPSYLHRAPSRIS |
| 45 | LPWRTSLLPSLPLRRRP |
| 46 | PPLFAKGPVGLLSRSFPP |
| 47 | VPPAPVVSLRSAHARPPY |
| 48 | LRPTPPRVRSYTCCPTP- |
| 49 | PNVAHVLPLLTVPWDNLR |
| 50 | CNPLLPLCARSPAVRTFP |

| | |
|---|---|
| (S) is optional in sequences 13 to 16. | |

Examples of rigid linkers include the peptide sequences GAPAPAAPAPA (SEQ ID NO:88), PPPP (SEQ ID NO:89) and PPP.

In one embodiment the peptide linker between the CH1 constant region fragment and the second variable domain in the heavy chain has the sequence given in SEQ ID NO:67 or SEQ ID NO:73.

In one embodiment the peptide linker between the CL domain and the second variable domain in the light chain has the sequence given in SEQ ID NO:67 or SEQ ID NO:73.

In one embodiment the peptide linker between the second variable domain in the heavy chain and the third variable domain in the heavy chain has the sequence given in SEQ ID NO:69 or SEQ ID NO:73.

In one embodiment the peptide linker between the second variable domain in the light chain and the third variable domain in the light chain has the sequence given in SEQ ID NO:69 or SEQ ID NO:73.
In one embodiment the peptide linker is an albumin binding peptide.
Examples of albumin binding peptides are provided in WO 2007/106120 and include:

**Table 3**

| **SEQ ID NO:** | **SEQUENCE** |
|---|---|
| 51 | DLCLRDWGCLW |
| 52 | DICLPRWGCLW |
| 53 | MEDICLPRWGCLWGD |
| 54 | QRLMEDICLPRWGCLWEDDE |
| 55 | QGLIGDICLPRWGCLWGRSV |
| 56 | QGLIGDICLPRWGCLWGRSVK |
| 57 | EDICLPRWGCLWEDD |
| 58 | RLMEDICLPRWGCLWEDD |
| 59 | MEDICLPRWGCLWEDD |
| 60 | MEDICLPRWGCLWED |
| 61 | RLMEDICLARWGCLWEDD |
| 62 | EVRSFCTRWPAEKSCKPLRG |
| 63 | RAPESFVCYWETICFERSEQ |
| 64 | EMCYFPGICWM |

In one embodiment the construct according to the present invention comprises an albumin binding peptide linked to the C-terminal thereof. This may be in addition or as an alternative to albumin binding peptide linkers.

In one embodiment at least one of the binding sites binds human serum albumin.

In one embodiment the first binding site binds human serum albumin.

In one embodiment the second binding site binds human serum albumin.

In one embodiment the third binding site binds human serum albumin

In one embodiment a variable domain in the heavy chain has the sequence given in SEQ ID NO:68. In one embodiment a variable domain in the light chain has the sequence given in SEQ ID NO:78.

It will be appreciated that one or more amino acid substitutions, additions and/or deletions may be made to the antibody variable domains, provided by the present invention, without significantly altering the ability of the antibody to bind to target antigen and to neutralise activity thereof. The effect of any amino acid substitutions, additions and/or deletions can be readily tested by one skilled in the art, for example by using the in vitro assays, for example a BIAcore assay.

In one embodiment the antibody heavy chain comprises a C_{H1} domain and the antibody light chain comprises a C_{L} domain, either kappa or lambda.

The antibody molecules of the present invention suitably have a high binding affinity, in particular nanomolar or picomolar. Affinity may be measured using any suitable method known in the art, including BIAcore. In one embodiment the antibody molecule of the present invention has a binding affinity of about 1nM or better or 100 pM or better. In one embodiment the antibody molecule of the present invention has a binding affinity of about 50pM or better. In one embodiment the antibody molecule of the present invention has a binding affinity of about 40pM or better. In one embodiment the antibody molecule of the present invention has a binding affinity of about 30pM or better. In one embodiment the antibody molecule of the present invention is fully human or humanised and has a binding affinity of about 100pM or better.

In one embodiment the antibody or heavy/light chain component thereof comprises the sequences given in SEQ ID NO:26 and SEQ ID NO:75. In one embodiment the antibody or heavy/light chain component thereof comprises the sequences given in SEQ ID NO:80 and SEQ ID NO:81. In one embodiment the antibody or heavy/light chain component thereof comprises the sequences given in SEQ ID NO:82 and SEQ ID NO:83. In one embodiment the antibody or heavy/light chain component thereof comprises the sequences given in SEQ ID NO:84 and SEQ ID NO:85. In one embodiment the antibody or heavy/light chain component thereof comprises the sequences given in SEQ ID NO:86 and SEQ ID NO:87.

If desired an antibody or heavy/light chain component thereof for use in the present invention may be conjugated to one or more effector molecule(s). It will be appreciated that the effector molecule may comprise a single effector molecule or two or more such molecules so linked as to form a single moiety that can be attached to the antibodies of the present invention. Where it is desired to obtain an antibody fragment linked to an effector molecule, this may be prepared by standard chemical or recombinant DNA procedures in which the antibody fragment is linked either directly or via a coupling agent to the effector molecule. Techniques for conjugating such effector molecules to antibodies are well known in the art (see, Hellstrom et al., Controlled Drug Delivery, 2nd Ed., Robinson et al., eds., 1987, pp. 623-53; Thorpe et al., 1982 , Immunol. Rev., 62:119-58 and Dubowchik et al., 1999, Pharmacology and Therapeutics, 83, 67-123). Particular chemical procedures include, for example, those described in WO 93/06231, WO 92/22583, WO 89/00195, WO 89/01476 and WO03031581. Alternatively, where the effector molecule is a protein or polypeptide the linkage may be achieved using recombinant DNA procedures, for example as described in WO 86/01533 and EP0392745.

The term effector molecule as used herein includes, for example, antineoplastic agents, drugs, toxins, biologically active proteins, for example enzymes, other antibody or antibody fragments, synthetic or naturally occurring polymers, nucleic acids and fragments thereof e.g. DNA, RNA and fragments thereof, radionuclides, particularly radioiodide, radioisotopes, chelated metals, nanoparticles and reporter groups such as fluorescent compounds or compounds which may be detected by NMR or ESR spectroscopy.

Examples of effector molecules may include cytotoxins or cytotoxic agents including any agent that is detrimental to (*e.g.* kills) cells. Examples include combrestatins, dolastatins, epothilones, staurosporin, maytansinoids, spongistatins, rhizoxin, halichondrins, roridins, hemiasterlins, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof.

Effector molecules also include, but are not limited to, antimetabolites (*e.g.* methotrexate, 6-mercaptopurine, 6-thioguanine, cytarabine, 5-fluorouracil decarbazine), alkylating agents (*e.g.* mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.* daunorubicin (formerly daunomycin) and doxorubicin), antibiotics (*e.g.* dactinomycin (formerly actinomycin), bleomycin, mithramycin, anthramycin (AMC), calicheamicins or duocarmycins), and anti-mitotic agents (*e.g.* vincristine and vinblastine).

Other effector molecules may include chelated radionuclides such as ¹¹¹In and ⁹⁰Y, Lu¹⁷⁷, Bismuth²¹³, Californium²⁵², Iridium¹⁹² and Tungsten¹⁸⁸/Rhenium¹⁸⁸; or drugs such as but not limited to, alkylphosphocholines, topoisomerase I inhibitors, taxoids and suramin.

Other effector molecules include proteins, peptides and enzymes. Enzymes of interest include, but are not limited to, proteolytic enzymes, hydrolases, lyases, isomerases, transferases. Proteins, polypeptides and peptides of interest include, but are not limited to, immunoglobulins, toxins such as abrin, ricin A, pseudomonas exotoxin, or diphtheria toxin, a protein such as insulin, tumour necrosis factor, α-interferon, β-interferon, nerve growth factor, platelet derived growth factor or tissue plasminogen activator, a thrombotic agent or an anti-angiogenic agent, *e.g.* angiostatin or endostatin, or, a biological response modifier such as a lymphokine, interleukin-1 (IL-1), interleukin-2 (IL-2), granulocyte macrophage colony stimulating factor (GM-CSF), granulocyte colony stimulating factor (G-CSF), nerve growth factor (NGF) or other growth factor and immunoglobulins.

Other effector molecules may include detectable substances useful for example in diagnosis. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc.

In another example the effector molecule may increase the half-life of the antibody *in vivo,* and/or reduce immunogenicity of the antibody and/or enhance the delivery of an antibody across an epithelial barrier to the immune system. Examples of suitable effector molecules of this type include polymers, albumin, albumin binding proteins or albumin binding compounds such as those described in WO05/117984.

Where the effector molecule is a polymer it may, in general, be a synthetic or a naturally occurring polymer, for example an optionally substituted straight or branched chain polyalkylene, polyalkenylene or polyoxyalkylene polymer or a branched or unbranched polysaccharide, e.g. a homo- or hetero- polysaccharide.

Specific optional substituents which may be present on the above-mentioned synthetic polymers include one or more hydroxy, methyl or methoxy groups.

Specific examples of synthetic polymers include optionally substituted straight or branched chain poly(ethyleneglycol), poly(propyleneglycol) poly(vinylalcohol) or derivatives thereof, especially optionally substituted poly(ethyleneglycol) such as methoxypoly(ethyleneglycol) or derivatives thereof.

Specific naturally occurring polymers include lactose, amylose, dextran, glycogen or derivatives thereof.

"Derivatives" as used herein is intended to include reactive derivatives, for example thiol-selective reactive groups such as maleimides and the like. The reactive group may be linked directly or through a linker segment to the polymer. It will be appreciated that the residue of such a group will in some instances form part of the product as the linking group between the antibody fragment and the polymer.

The size of the polymer may be varied as desired, but will generally be in an average molecular weight range from 500Da to 50000Da, for example from 5000 to 40000Da such as from 20000 to 40000Da. The polymer size may in particular be selected on the basis of the intended use of the product for example ability to localize to certain tissues such as tumors or extend circulating half-life (for review see Chapman, 2002, Advanced Drug Delivery Reviews, 54, 531-545). Thus, for example, where the product is intended to leave the circulation and penetrate tissue, for example for use in the treatment of a tumour, it may be advantageous to use a small molecular weight polymer, for example with a molecular weight of around 5000Da. For applications where the product remains in the circulation, it may be advantageous to use a higher molecular weight polymer, for example having a molecular weight in the range from 20000Da to 40000Da.

Suitable polymers include a polyalkylene polymer, such as a poly(ethyleneglycol) or, especially, a methoxypoly(ethyleneglycol) or a derivative thereof, and especially with a molecular weight in the range from about 15000Da to about 40000Da.

In one example antibodies for use in the present invention are attached to poly(ethyleneglycol) (PEG) moieties. In one particular example the antibody is an antibody fragment and the PEG molecules may be attached through any available amino acid side-chain or terminal amino acid functional group located in the antibody fragment, for example any free amino, imino, thiol, hydroxyl or carboxyl group. Such amino acids may occur naturally in the antibody fragment or may be engineered into the fragment using recombinant DNA methods (see for example US 5,219,996; US 5,667,425; WO98/25971). In one example the antibody molecule of the present invention is a modified Fab fragment wherein the modification is the addition to the C-terminal end of its heavy chain one or more amino acids to allow the attachment of an effector molecule. Suitably, the additional amino acids form a modified hinge region containing one or more cysteine residues to which the effector molecule may be attached. Multiple sites can be used to attach two or more PEG molecules.

In one embodiment a PEG molecule is linked to a cysteine 171 in the light chain, for example see WO2008/038024 incorporated herein by reference.

Suitably PEG molecules are covalently linked through a thiol group of at least one cysteine residue located in the antibody fragment. Each polymer molecule attached to the modified antibody fragment may be covalently linked to the sulphur atom of a cysteine residue located in the fragment. The covalent linkage will generally be a disulphide bond or, in particular, a sulphur-carbon bond. Where a thiol group is used as the point of attachment appropriately activated effector molecules, for example thiol selective derivatives such as maleimides and cysteine derivatives may be used. An activated polymer may be used as the starting material in the preparation of polymer-modified antibody fragments as described above. The activated polymer may be any polymer containing a thiol reactive group such as an α-halocarboxylic acid or ester, e.g. iodoacetamide, an imide, e.g. maleimide, a vinyl sulphone or a disulphide. Such starting materials may be obtained commercially (for example from Nektar, formerly Shearwater Polymers Inc., Huntsville, AL, USA) or may be prepared from commercially available starting materials using conventional chemical procedures. Particular PEG molecules include 20K methoxy-PEG-amine (obtainable from Nektar, formerly Shearwater; Rapp Polymere; and SunBio) and M-PEG-SPA (obtainable from Nektar, formerly Shearwater).

The present invention also provides isolated DNA encoding an antibody described herein or a fragment thereof of a heavy or light chain thereof.

In a further aspect there is provided a vector comprising said DNA.

General methods by which the vectors may be constructed, transfection methods and culture methods are well known to those skilled in the art. In this respect, reference is made to "Current Protocols in Molecular Biology", 1999, F. M. Ausubel (ed), Wiley Interscience, New York and the Maniatis Manual produced by Cold Spring Harbor Publishing.

In a further aspect there is provided a host cell comprising said vector and/or DNA.

Any suitable host cell/vector system may be used for expression of the DNA sequences encoding the antibody molecule of the present invention. Bacterial, for example *E. coli,* and other microbial systems may be used or eukaryotic, for example mammalian, host cell expression systems may also be used. Suitable mammalian host cells include CHO, myeloma or hybridoma cells.

The present invention also provides a process for the production of an antibody molecule according to the present invention comprising culturing a host cell containing a vector (and/or DNA) of the present invention under conditions suitable for leading to expression of protein from DNA encoding the antibody molecule of the present invention, and isolating the antibody molecule.

The antibody molecule may comprise only a heavy or light chain polypeptide, in which case only a heavy chain or light chain polypeptide coding sequence needs to be used to transfect the host cells. For production of products comprising both heavy and light chains, the cell line may be transfected with two vectors, a first vector encoding a light chain polypeptide and a second vector encoding a heavy chain polypeptide. Alternatively, a single vector may be used, the vector including sequences encoding light chain and heavy chain polypeptides.

The antibodies and fragments according to the present disclosure are expressed at good levels from host cells. Thus the properties of the antibodies and/or fragments are conducive to commercial processing.

The antibodies of the present invention are useful in the treatment and/or prophylaxis of a pathological condition.

Thus there is provided an antibody or single chain component thereof for use in treatment, by administering a therapeutically effective amount thereof. In one embodiment the antibody or single chain component thereof is administered in as a pharmaceutical formulation.

Thus the present invention also provides a pharmaceutical or diagnostic composition comprising an antibody molecule of the present invention in combination with one or more of a pharmaceutically acceptable excipient, diluent or carrier. Accordingly, provided is the use of an antibody of the invention for the manufacture of a medicament. The composition will usually be supplied as part of a sterile, pharmaceutical composition that will normally include a pharmaceutically acceptable carrier. A pharmaceutical composition of the present invention may additionally comprise a pharmaceutically-acceptable adjuvant.

The present invention also provides a process for preparation of a pharmaceutical or diagnostic composition comprising adding and mixing the antibody molecule of the present invention together with one or more of a pharmaceutically acceptable excipient, diluent or carrier.

The antibody molecule may be the sole active ingredient in the pharmaceutical or diagnostic composition or may be accompanied by other active ingredients including other antibody ingredients, for example anti-TNF, anti- IL-1β, anti-T cell, anti-IFNγ or anti-LPS antibodies, or non-antibody ingredients such as xanthines. Other suitable active ingredients include antibodies capable of inducing tolerance, for example, anti-CD3 or anti-CD4 antibodies.

In a further embodiment the antibody, fragment or composition according to the disclosure is employed in combination with a further pharmaceutically active agent, for example a corticosteroid (such as fluticasonoe propionate) and/or a beta-2-agonist (such as salbutamol, salmeterol or formoterol) or inhibitors of cell growth and proliferation (such as rapamycin, cyclophosphmide, methotrexate) or alternative a CD28 and /or CD40 inhibitor. In one embodiment the inhitor is a small molecule. In another embodiment the inhibitor is an antibody specific to the target.

The pharmaceutical compositions suitably comprise a therapeutically effective amount of the antibody of the invention. The term "therapeutically effective amount" as used herein refers to an amount of a therapeutic agent needed to treat, ameliorate or prevent a targeted disease or condition, or to exhibit a detectable therapeutic or preventative effect. For any antibody, the therapeutically effective amount can be estimated initially either in cell culture assays or in animal models, usually in rodents, rabbits, dogs, pigs or primates. The animal model may also be used to determine the appropriate concentration range and route of administration. Such information can then be used to determine useful doses and routes for administration in humans.

The precise therapeutically effective amount for a human subject will depend upon the severity of the disease state, the general health of the subject, the age, weight and gender of the subject, diet, time and frequency of administration, drug combination(s), reaction sensitivities and tolerance/response to therapy. This amount can be determined by routine experimentation and is within the judgement of the clinician. Generally, a therapeutically effective amount will be from 0.01 mg/kg to 50 mg/kg, for example 0.1 mg/kg to 20 mg/kg. Pharmaceutical compositions may be conveniently presented in unit dose forms containing a predetermined amount of an active agent of the invention per dose.

Compositions may be administered individually to a patient or may be administered in combination (*e.g.* simultaneously, sequentially or separately) with other agents, drugs or hormones.

The dose at which the antibody molecule of the present invention is administered depends on the nature of the condition to be treated, the extent of the inflammation present and on whether the antibody molecule is being used prophylactically or to treat an existing condition.

The frequency of dose will depend on the half-life of the antibody molecule and the duration of its effect. If the antibody molecule has a short half-life (e.g. 2 to 10 hours) it may be necessary to give one or more doses per day. Alternatively, if the antibody molecule has a long half life (e.g. 2 to 15 days) it may only be necessary to give a dosage once per day, once per week or even once every 1 or 2 months.

The pharmaceutically acceptable carrier should not itself induce the production of antibodies harmful to the individual receiving the composition and should not be toxic. Suitable carriers may be large, slowly metabolised macromolecules such as proteins, polypeptides, liposomes, polysaccharides, polylactic acids, polyglycolic acids, polymeric amino acids, amino acid copolymers and inactive virus particles.

Pharmaceutically acceptable salts can be used, for example mineral acid salts, such as hydrochlorides, hydrobromides, phosphates and sulphates, or salts of organic acids, such as acetates, propionates, malonates and benzoates.

Pharmaceutically acceptable carriers in therapeutic compositions may additionally contain liquids such as water, saline, glycerol and ethanol. Additionally, auxiliary substances, such as wetting or emulsifying agents or pH buffering substances, may be present in such compositions. Such carriers enable the pharmaceutical compositions to be formulated as tablets, pills, dragees, capsules, liquids, gels, syrups, slurries and suspensions, for ingestion by the patient.

Suitable forms for administration include forms suitable for parenteral administration, e.g. by injection or infusion, for example by bolus injection or continuous infusion. Where the product is for injection or infusion, it may take the form of a suspension, solution or emulsion in an oily or aqueous vehicle and it may contain formulatory agents, such as suspending, preservative, stabilising and/or dispersing agents. Alternatively, the antibody molecule may be in dry form, for reconstitution before use with an appropriate sterile liquid.

Once formulated, the compositions of the invention can be administered directly to the subject. The subjects to be treated can be animals. However, in one or more embodiments the compositions are adapted for administration to human subjects.

Suitably in formulations according to the present disclosure, the pH of the final formulation is not similar to the value of the isoelectric point of the antibody or fragment, for example if the pH of the formulation is 7 then a pI of from 8-9 or above may be appropriate.

Whilst not wishing to be bound by theory it is thought that this may ultimately provide a final formulation with improved stability, for example the antibody or fragment remains in solution.

The pharmaceutical compositions of this invention may be administered by any number of routes including, but not limited to, oral, intravenous, intramuscular, intra-arterial, intramedullary, intrathecal, intraventricular, transdermal, transcutaneous (for example, see WO98/20734), subcutaneous, intraperitoneal, intranasal, enteral, topical, sublingual, intravaginal or rectal routes. Hyposprays may also be used to administer the pharmaceutical compositions of the invention. Typically, the therapeutic compositions may be prepared as injectables, either as liquid solutions or suspensions. Solid forms suitable for solution in, or suspension in, liquid vehicles prior to injection may also be prepared.

Direct delivery of the compositions will generally be accomplished by injection, subcutaneously, intraperitoneally, intravenously or intramuscularly, or delivered to the interstitial space of a tissue. The compositions can also be administered into a lesion. Dosage treatment may be a single dose schedule or a multiple dose schedule.

It will be appreciated that the active ingredient in the composition will be an antibody molecule. As such, it will be susceptible to degradation in the gastrointestinal tract. Thus, if the composition is to be administered by a route using the gastrointestinal tract, the composition will need to contain agents which protect the antibody from degradation but which release the antibody once it has been absorbed from the gastrointestinal tract.

A thorough discussion of pharmaceutically acceptable carriers is available in Remington's Pharmaceutical Sciences (Mack Publishing Company, N.J. 1991).

In one embodiment the formulation is provided as a formulation for topical administrations including inhalation.

Suitable inhalable preparations include inhalable powders, metering aerosols containing propellant gases or inhalable solutions free from propellant gases. Inhalable powders according to the disclosure containing the active substance may consist solely of the abovementioned active substances or of a mixture of the abovementioned active substances with physiologically acceptable excipient.

These inhalable powders may include monosaccharides (e.g. glucose or arabinose), disaccharides (e.g. lactose, saccharose, maltose), oligo- and polysaccharides (e.g. dextranes), polyalcohols (e.g. sorbitol, mannitol, xylitol), salts (e.g. sodium chloride, calcium carbonate) or mixtures of these with one another. Mono- or disaccharides are suitably used, the use of lactose or glucose, particularly but not exclusively in the form of their hydrates.

Particles for deposition in the lung require a particle size less than 10 microns, such as 1-9 microns for example from 0.1 to 5 µm, in particular from 1 to 5 µm. The particle size of the active ingredient (such as the antibody or fragment) is of primary importance.

The propellent gases which can be used to prepare the inhalable aerosols are known in the art. Suitable propellent gases are selected from among hydrocarbons such as n-propane, n-butane or isobutane and halohydrocarbons such as chlorinated and/or fluorinated derivatives of methane, ethane, propane, butane, cyclopropane or cyclobutane. The abovementioned propellent gases may be used on their own or in mixtures thereof.

Particularly suitable propellent gases are halogenated alkane derivatives selected from among TG 11, TG 12, TG 134a and TG227. Of the abovementioned halogenated hydrocarbons, TG134a (1,1,1,2-tetrafluoroethane) and TG227 (1,1,1,2,3,3,3-heptafluoropropane) and mixtures thereof are particularly suitable.

The propellent-gas-containing inhalable aerosols may also contain other ingredients such as cosolvents, stabilisers, surface-active agents (surfactants), antioxidants, lubricants and means for adjusting the pH. All these ingredients are known in the art.

The propellant-gas-containing inhalable aerosols according to the invention may contain up to 5 % by weight of active substance. Aerosols according to the invention contain, for example, 0.002 to 5 % by weight, 0.01 to 3 % by weight, 0.015 to 2 % by weight, 0.1 to 2 % by weight, 0.5 to 2 % by weight or 0.5 to 1 % by weight of active ingredient.

Alternatively topical administrations to the lung may also be by administration of a liquid solution or suspension formulation, for example employing a device such as a nebulizer, for example, a nebulizer connected to a compressor (e.g., the Pari LC-Jet Plus(R) nebulizer connected to a Pari Master(R) compressor manufactured by Pari Respiratory Equipment, Inc., Richmond, Va.).

The antibody of the invention can be delivered dispersed in a solvent, e.g., in the form of a solution or a suspension. It can be suspended in an appropriate physiological solution, e.g., saline or other pharmacologically acceptable solvent or a buffered solution. Buffered solutions known in the art may contain 0.05 mg to 0.15 mg disodium edetate, 8.0 mg to 9.0 mg NaCl, 0.15 mg to 0.25 mg polysorbate, 0.25 mg to 0.30 mg anhydrous citric acid, and 0.45 mg to 0.55 mg sodium citrate per 1 ml of water so as to achieve a pH of about 4.0 to 5.0. A suspension can employ, for example, lyophilised antibody.

The therapeutic suspensions or solution formulations can also contain one or more excipients. Excipients are well known in the art and include buffers (e.g., citrate buffer, phosphate buffer, acetate buffer and bicarbonate buffer), amino acids, urea, alcohols, ascorbic acid, phospholipids, proteins (e.g., serum albumin), EDTA, sodium chloride, liposomes, mannitol, sorbitol, and glycerol. Solutions or suspensions can be encapsulated in liposomes or biodegradable microspheres. The formulation will generally be provided in a substantially sterile form employing sterile manufacture processes.

This may include production and sterilization by filtration of the buffered solvent/solution used for the formulation, aseptic suspension of the antibody in the sterile buffered solvent solution, and dispensing of the formulation into sterile receptacles by methods familiar to those of ordinary skill in the art.

Nebulizable formulation according to the present disclosure may be provided, for example, as single dose units (e.g., sealed plastic containers or vials) packed in foil envelopes. Each vial contains a unit dose in a volume, e.g., 2 ml, of solvent/solution buffer.

The antibodies of the present disclosure are thought to be suitable for delivery via nebulisation.

Comprising in the context of the present specification is intended to meaning including.

Where technically appropriate embodiments of the invention may be combined.

Embodiments are described herein as comprising certain features/elements. The disclosure also extends to separate embodiments consisting or consisting essentially of said features/elements.

The present invention is further described by way of illustration only in the following examples, which refer to the accompanying Figures, in which:

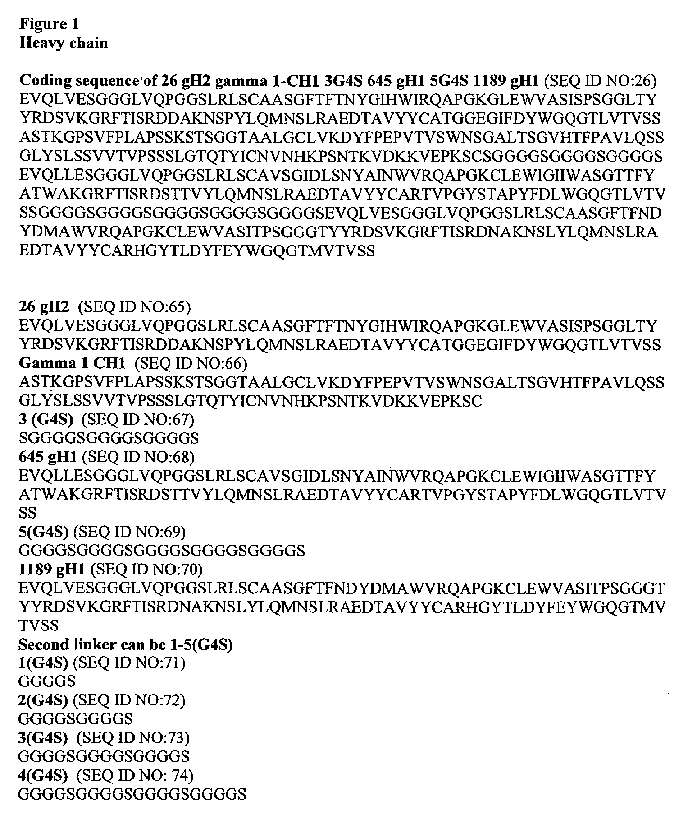

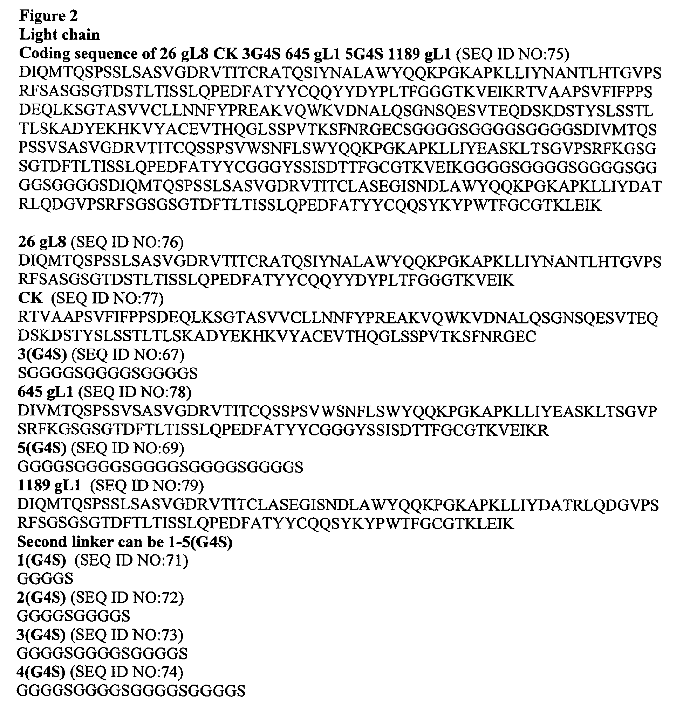

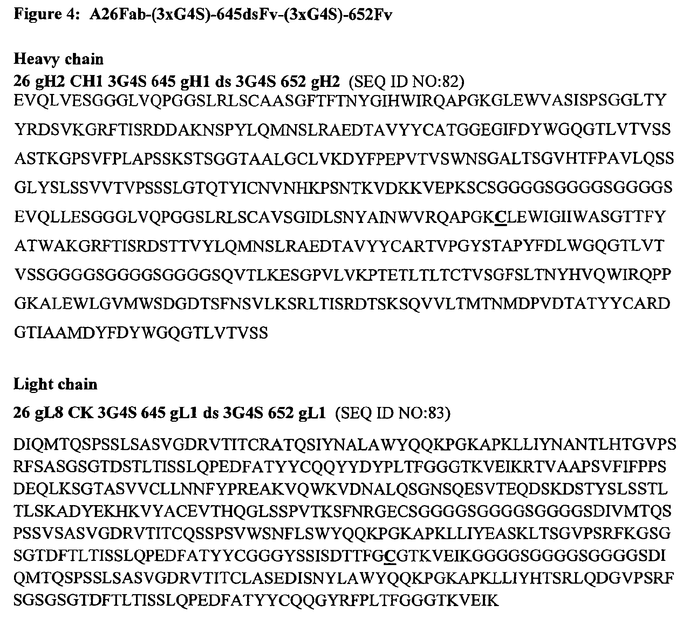

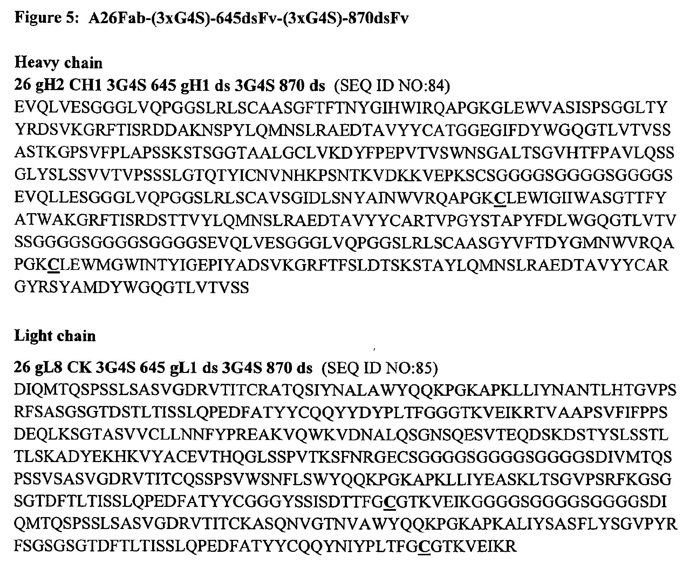

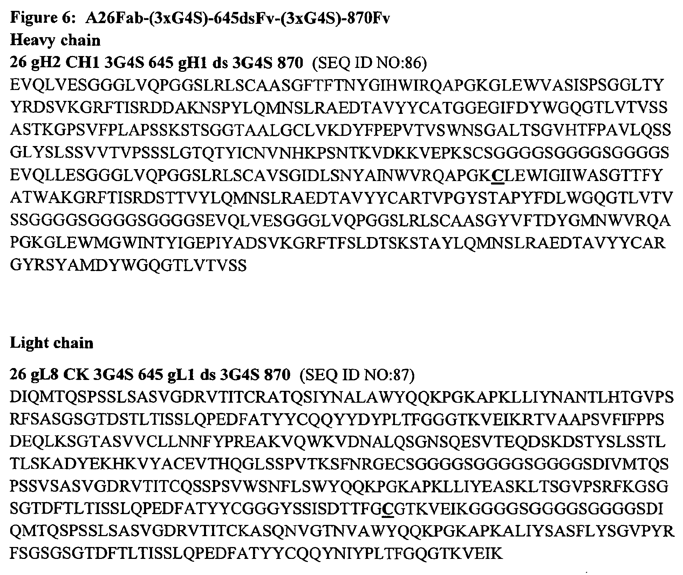

Figures 1-6 show antibody or heavy/light chain component sequences according to the present invention.

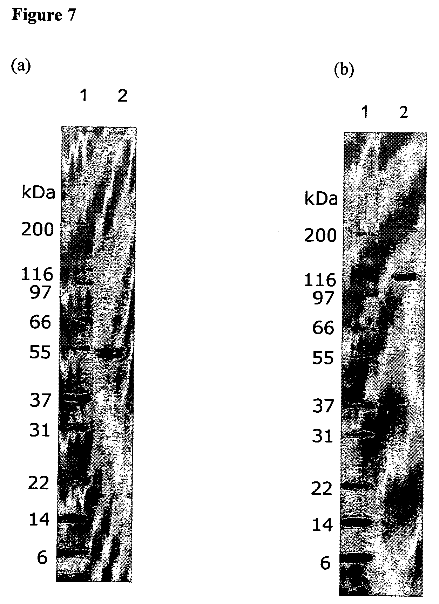

Figure 7 shows SDS-PAGE analysis of FabA-(3xG4S)-645dsFv-(3xG4S)-652dsFv) under (a) reducing conditions and (b) non-reducing conditions.

### EXAMPLES

### Example 1: Generation of Antibody FabFvFv

The FabFvFv was generated by overlappping PCR method that linked an existing 26 gH2 Fab 3G4S 645 gH1 coding region to 1-5(G4S)1189 gH1. The junction being the end of 645 gH1 and 1-5(G4S). The above coding region was cloned into our standard UCB mammalian expression vector under the control of the HCMV-MIE promoter and SV40E polyA sequence. The DNA was paired with a similar plasmid encoding the corresponding light chain based on the second linker length (26 gL8 CK 3G4S 645 gL1 1-5G4S 1189 gLl) and used to transfect HEK293 cells in 6-well dishes. Invitrogen's 293fectin was used to transfect the cells and then the cells were incubated for 6 days on a shaking platform at 37°C. Supernatants were harvested and the amount of secreted antibody quantified by ELISA. The supernatants were then submitted for BIAcore analysis, results of which are shown in Table 4. The sequences for the antibody are shown in Figures 1-3.

### BIAcore Assay

A26Fab is specific for OX40, 645Fv has specificity for human serum albumin (HSA) and 1189 has specificity for IL-6.

Binding affinities and kinetic parameters for the interaction of A26Fab-645Fv-1189Fv with HSA (Jackson ImmunoResearch, 009-000-051) and IL-6 were determined by surface plasmon resonance (SPR) conducted on a Biacore T100 using CM5 sensor chips and HBS-EP (10mM HEPES (pH7.4), 150mM NaCl, 3mM EDTA, 0.05% v/v surfactant P20) running buffer. The A26Fab-645Fv-1189Fv samples were captured to the sensor chip surface using an in-house generated anti-human CH1 monoclonal antibody. Covalent immobilisation of the capture antibody was achieved by standard amine coupling chemistry.

Each assay cycle consisted of firstly capturing the A26Fab-645Fv-1189Fv using a 1 min injection, before an association phase consisting of a 3 min injection of antigen, after which dissociation was monitored for 8 min. After each cycle, the capture surface was regenerated with 2 x 1 min injections of 40mM HC1 followed by 30s of 5mM NaOH. The flow rates used were 10µl/min for capture, 30µl/min for association and dissociation phases, and 10µl/min for regeneration.

Titrations of human serum albumin were performed at concentrations of 250, 125, 62.5 and 31.25µM, Il-6 titrations were performed at concentrations of 10, 5, 2.5, 1.25 and 0.625nM. A blank flow-cell was used for reference subtraction and buffer-blank injections were included to subtract instrument noise and drift.

Kinetic parameters were determined by simultaneous global-fitting of the resulting sensorgrams to a standard 1:1 binding model using Biacore T100 Evaluation Software v2.0.1.

**Table 4: Summary of HSA and IL-6 binding to Tri-specific constructs**

| **Affinity for HSA** | | | | |
|---|---|---|---|---|
| **Sample** | **ka (1/Ms)** | **kd** (**1**/**s**) | **KD (nM)** | **Stoichiometry** |
| **A26Fab 645Fv** | 4.69E+04 | 2.69E-04 | **5.74** | 0.58 |
| **A26Fab 645Fv 1G4S 1189Fv** | 3.34E+04 | 3.79E-04 | **11.33** | 0.39 |
| **A26Fab 645Fv 2G4S 1189Fv** | 3.37E+04 | 3.89E-04 | **11.55** | 0.40 |
| **A26Fab 645Fv 3G4S 1189Fv** | 3.38E+04 | 3.92E-04 | **11.60** | 0.39 |
| **A26Fab 645Fv 4G4S 1189Fv** | 3.39E+04 | 3.96E-04 | **11.66** | 0.40 |
| **A26Fab 645Fv 5G4S 1189Fv** | 3.24E+04 | 3.93E-04 | **12.15** | 0.39 |
| | | | | |

| **Affinity for IL-6** | | | | |
|---|---|---|---|---|
| **Sample** | **ka (1/Ms)** | **kd (1/s)** | **KD (nM)** | **Stoichiometry** |
| **1189 IgG** | 7.68E+06 | 4.90E-04 | **0.06** | 0.84 |
| **A26Fab 645Fv 1G4S 1189Fv** | 1 RU of binding | | | 0.06 |
| **A26Fab 645Fv 2G4S 1189Fv** | 9.59E+05 | 7.47E-04 | **0.78** | 0.47 |
| **A26Fab 645Fv 3G4S 1189Fv** | 8.74E+05 | 7.37E-04 | **0.84** | 0.68 |
| **A26Fab 645Fv 4G4S 1189Fv** | 1.41E+06 | 7.29E-04 | **0.52** | 0.66 |
| **A26Fab 645Fv 5G4S 1189Fv** | 1.61E+06 | 7.68E-04 | **0.48** | 0.69 |

### Example 2

### A26Fab-(3xG4S)-645dsFv-(3xG4S)-652dsFv

### A26Fab-(3xG4S)-645dsFv-(3xG4S)-652Fv

### A26Fab-(3xG4S)-645dsFv-(3xG4S)-870dsFv

### A26Fab-(3xG4S)-645dsFv-(3xG4S)-870Fv

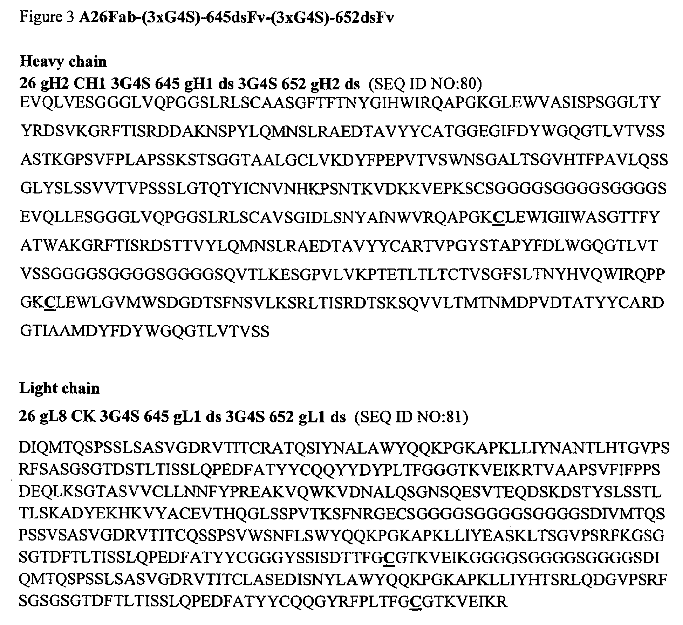

The heavy chain of the FabFvFv was generated by overlapping PCR method that linked an existing 26 Fab 3G4S 645 Fv heavy chain coding region to either 3G4S 652 gH1 or 3G4S 870H. Similarly an existing 26 Fab 3G4S 645 Fv light chain coding region was linked to either 3G4S 652 gL2 or 3G4S 870k. The junction being the end of 645 Fv and the second 3G4S. The sequences are provided in Figures 3-6. The coding regions were then cloned into our standard UCB mammalian expression vector under the control of the HCMV-MIE promoter and SV40E polyA sequence. The heavy and light chain DNA plasmids were paired appropriately and used to transfect HEK293 cells in 6-well dishes. Invitrogen's 293fectin was used to transfect the cells and then the cells were incubated for 6 days on a shaking platform at 37°C. Supernatants were harvested and the amount of secreted antibody quantified by ELISA. The supernatants were then submitted for BIAcore analysis.

Binding affinities and kinetic parameters for the interaction of A26Fab-645Fv-652/870Fv with IL13 (R&D 213IL) and TNF (Strathmann hTNFa) were determined by surface plasmon resonance (SPR) conducted on a Biacore 3000 using CM5 sensor chips and HBS-EP (10mM HEPES (pH7.4), 150mM NaCl, 3mM EDTA, 0.005% v/v surfactant P20) running buffer. The A26Fab-645Fv-652/870Fv samples were captured to the sensor chip surface using an in-house generated anti-human CH1 monoclonal antibody. Covalent immobilisation of the capture antibody was achieved by standard amine coupling chemistry.

Each assay cycle consisted of firstly capturing the A26Fab-645Fv-652/870Fv using a 1 min injection, before an association phase consisting of a 3 min injection of antigen, after which dissociation was monitored for 15 min. After each cycle, the capture surface was regenerated with 2 x 1 min injections of 40mM HCl followed by 30s of 5mM NaOH. The flow rates used were 10µl/min for capture, 30µl/min for association and dissociation phases, and 10µl/min for regeneration.

Titrations of IL 13 were performed at concentrations of 20, 10, 5, 2.5 and 1.25nM, TNF titrations were performed at concentrations of 10, 5, 2.5, 1.25 and 0.625nM. A blank flow-cell was used for reference subtraction and buffer-blank injections were included to subtract instrument noise and drift.
Kinetic parameters were determined by simultaneous global-fitting of the resulting sensorgrams to a standard 1:1 binding model using Biacore 3000 Evaluation Software v3.2

**Table 5. Antigen binding as determined by BIAcore**

| **Sample** | **Antigen** | **kDa** | **Affinity** | **Stoichiometry** |
|---|---|---|---|---|
| **652 IgG** | IL13 | 12.5 | Retained | 0.99 |
| **A26-645-652+DS** | IL13 | 12.5 | 2 fold loss | 0.88 |
| **A26-645-652-DS** | IL13 | 12.5 | Retained | 0.95 |
| **870 Fab** | TNF | 54 | Retained | 0.63 |
| **A26-645-870+DS** | TNF | 54 | 2 fold loss | 0.42 |
| **A26-645-870-DS** | TNF | 54 | 2 fold loss | 0.39 |

Table 5 shows that all of the terminal v-regions have affinities that are within 2 fold of the parent Fab. They also have stoichiometries that are greater than 0.4. This demonstrates that the terminal v-regions have good binding characteristics for their respective antigens.

SPR was also used to determine the binding of multiple antigens at once to the A26Fab-645Fv-652/870Fv samples on a Biacore 3000 using CM5 sensor chips and HBS-EP (10mM HEPES (pH7.4), 150mM NaCl, 3mM EDTA, 0.005% v/v surfactant P20) running buffer. The A26Fab-645Fv-652/870Fv samples were captured to the sensor chip surface using an in-house generated anti-human CH1 monoclonal antibody. Covalent immobilisation of the capture antibody was achieved by standard amine coupling chemistry.

Each assay cycle consisted of firstly capturing the A26Fab-645Fv-652/870Fv using a 1 min injection, before an association phase consisting of a 3 min injection of antigen(s), after which dissociation was monitored for 15 min. After each cycle, the capture surface was regenerated with 2 x 1 min injections of 40mM HCl followed by 30s of 5mM NaOH. The flow rates used were 10µl/min for capture, 30µl/min for association and dissociation phases, and 10µl/min for regeneration.

The binding response of individual antigens at a single concentration (20nM Ox40/mFc (AXXORA 1588); 50nM HSA (Jackson ImmunoResearch, 009-000-051); 20nM IL13 (R&D 213IL) and 10nM TNF (Strathmann hTNFa)) to A26Fab-645Fv-652/870Fv was measured in separate cycles. The binding response of antigen mixtures with the same final concentration of each specific antigen were also measured (20nM IL13/ 50nM HSA; 20nM IL13/50nM HSA/20nM 0x40; 10nM TNF/50nM HSA a 10nM TNF/50nM HSA/20nM Ox40). As the concentration of each antigen is the same in the mixture as it is individually the response for each antigen component should be the same.

**Table 6: Binding response data**

| | | | **Sum of individual binding responses** | |
|---|---|---|---|---|
| **Constructs** | **Analyte** | **Response RU** | **HSA+TNF** | **HSA+TNF+OX40** |
| **A26-645-870+DS** | OX40 | 32 | | |
| | HSA | 10 | | |
| | TNF | 22 | | |
| | HSA TNF | 32 | 32 | |
| | HSA TNF OX40 | 63 | | 64 |
| **A26**-**645-870-DS** | OX40 | 31 | | |
| | HSA | 11 | | |
| | TNF | 21 | | |
| | HSA TNF | 33 | 32 | |
| | HSA TNF OX40 | 62 | | 63 |

| **Constructs** | **Analyte** | **Response RU** | **HSA+IL13** | **HSA+IL13+OX40** |
|---|---|---|---|---|
| **A26-645 -652+DS** | OX40 | 39 | | |
| | HSA | 11 | | |
| | IL13 | 15 | | |
| | HSA IL13 | 24 | 26 | |
| | HSA IL13 OX40 | 65 | | 65 |
| **A26-645 -652**-**DS** | OX40 | 42 | | |
| | HSA | 12 | | |
| | IL13 | 17 | | |
| | HSA IL13 | 29 | 29 | |
| | HSA_IL13_OX40 | 71 | | 71 |

By comparing the response units for the binding of the individual antigens to the response units when antigens are added at the same time the effect of binding of any one antigen on the binding of the other antigens can be assessed. As the sum of the binding for the individual antigens is the same as the antigens applied at the same time then concurrent binding of all 3 antigens happens (Table 6). All of the v-regions are capable of binding their antigens to the same extent regardless of the presence of antigens on any of the other v-regions.

### Example 3

### FabA-(3xG4S)-645dsFv-(3xG4S)-652dsFv

The FabAFvFv was derived from previously generated constructs using overlapping PCR.

### Expression and purification of FabA-(3xG4S)-645dsFv-(3xG4S)-652dsFv

### Mammalian expression

HEK293 cells were transfected with the heavy and light chain plasmids using Invitrogen's 293fectin transfection reagent according to the manufacturer's instructions. Briefly, 2µg heavy chain plasmid and 2µg light chain plasmid were incubated with 10µl 293fectin and 340µl Optimem media for 20mins at RT. The mixture was then added to 5x10⁶ HEK293 cells in suspension and incubated for 7 days with shaking at 37°C. After 7 days the supernatant was collected by centrifugation at 1500xg to remove the cells and then 0.22µm sterile filtered. The expression level was determined by Protein-G assay to be 7.4µg/ml

### Protein-G purification

The mammalian supernatant containing FabA-(3xG4S)-645dsFv-(3xG4S)-652dsFv was concentrated from ∼45ml to ∼2ml using an Amicon Ultra-15 concentrator with a 10kDa molecular weight cut off membrane and centrifugation at 4000xg in a swing out rotor. 1.8ml of the concentrated supernatant was applied at 1ml/min to a 1ml HiTrap Protein-G FF (GE Healthcare) column equilibrated in 20mM phosphate, 150mM NaCl pH7.4. The column was washed with 20mM phosphate, 150mM NaCl pH7.4 and the bound material eluted with 0.1M glycine/HCl pH2.7. The elution peak was collected and pH adjusted to ∼pH7 with 2M Tris/HCl pH8.8. The pH adjusted elution was diafiltered into 20mM phosphate, 150mM NaCl pH7.4 and concentrated to ∼0.5ml using an Amicon Ultra-15 concentrator with a 10kDa molecular weight cut off membrane and centrifugation at 4000xg in a swing out rotor. The concentration of the purified FabA-(3xG4S)-645dsFv-(3xG4S)-652dsFv was determined by absorbance at 280nm to be 72µg/ml

### SDS-PAGE analysis of FabA-(3xG4S)-645dsFv-(3xG4S)-652dsFv

To 26µl of the purified FabA-(3xG4S)-645dsFv-(3xG4S)-652dsFv was added 10µL 4X LDS (Invitrogen) sample running buffer. For the non-reduced sample, 4µL of 100mM NEM was added and for the reduced sample 4µL of 10X reducing agent (Invitrogen) was added. The samples were vortexed, incubated at 100oC for 3 mins, cooled and centrifuged at 12500 rpm for 30secs. 30µl of the prepared samples were loaded on to a 4-20% acrylamine Tris/Glycine SDS gel and run for 110mins at 125V. The gels were stained with Coomassie Blue protein stain and destained with 7.5% acetic acid. Under reducing conditions, see Figure 7(a), the FabA-(3xG4S)-645dsFv-(3xG4S)-652dsFv is essentially 2 band of ∼55kDa, one for each chain. Under non-reducing conditions, see Figure 7(b), the main band at ∼116kDa is FabA-(3xG4S)-645dsFv-(3xG4S)-652dsFv monomer whereas the bands above are various forms of multimer of FabA-(3xG4S)-645dsFv-(3xG4S)-652dsFv.

## Claims

1. A recombinant antibody or a heavy/light chain component thereof comprising:
a heavy chain comprising a C_{H1} constant region fragment located between a first and
second variable domain, and a third variable domain linked directly or indirectly to the first or second variable domain,
with the proviso that the heavy chain only contains one C_{H1} and only contains three variable domains, and
a light chain comprising at least a C_{L} domain located between a first and second variable domain, and a third variable domain linked directly or indirectly to the first or
second variable domain,
with the proviso that the light chain only contains one C_{L} domain and only contains three variable domains, and
wherein said heavy and light chains are aligned to provide a first binding site formed by the first variable domains in the light and heavy chain, a second binding site formed by the second variable domains in the light and heavy chain, and a third binding site formed by the third variable domains in the light and heavy chain.

2. A recombinant antibody or a heavy/light chain component thereof according to claim 1 wherein C_{H1} is fused to the first variable domain in the heavy chain.

3. A recombinant antibody or a heavy/light chain component thereof according to claim 1 or 2 wherein C_{L} is fused to the first variable domain in the light chain.

4. A recombinant antibody or a heavy/light chain component thereof according to any one of claims 1 to 3, wherein C_{H1} is linked indirectly to the second variable domain in the heavy chain.

5. A recombinant antibody or a heavy/light chain component thereof according to any one of claims 1 to 4, wherein C_{L} is linked indirectly to the second variable domain in the light chain.

6. A recombinant antibody or a heavy/light chain component thereof according to claim 4 or claim 5 wherein the indirect link is a peptide linker.

7. A recombinant antibody or a heavy/light chain component thereof according to any one of claims 1 to 6, wherein the third variable domain is linked indirectly.

8. A recombinant antibody or a heavy/light chain component thereof according to claim 7 wherein the indirect link is a peptide linker.

9. A recombinant antibody or a heavy/light chain component thereof according to any one of claims 1 to 8, wherein the third variable domain in the heavy and/or light chain is linked to the first variable domain therein.

10. A recombinant antibody or a heavy/light chain component thereof according to any one of claims 1 to 8, wherein the third variable domain in the heavy and/or light chain is linked to the second variable domain therein.

11. A recombinant antibody or a heavy/light chain component thereof according to any one of claims 1 to 10 wherein each binding site is formed by a variable domain VH/VL pair which is a cognate pair.

12. A recombinant antibody or a heavy/light chain component thereof according to any one of claims 1 to 11 wherein each binding site is formed by a variable domain VH/VL pair which are a complementary VH/VL pair which bind antigen co-operatively.

13. A recombinant antibody or a heavy/light chain component thereof according to any one of claims 1 to 12, wherein the variable domains of at least one binding site are linked by a disulfide bond.

14. A recombinant antibody or heavy/light chain component thereof according to any one of claims 1 to 13 wherein the second variable domains forming the second binding site are stabilized by a disulfide bond.

15. A recombinant antibody or a heavy/light chain component thereof according to any one of claims 1 to 14, wherein third variable domains forming the third binding site are stabilized by a disulfide bond.

16. A recombinant antibody or a heavy/light chain component thereof according to any one of claims 1 to 15, wherein the C_{H1} of the heavy chain is linked to the C_{L} of the light chain by a disulfide bond.

17. A recombinant protein according to claim 16, where in C_{H1} does not comprise a hinge region.

18. A recombinant antibody or a heavy/light chain component thereof according to any one of claims 1 to 17 which is monospecific, bispecific or trispecific.

## Patentansprüche

1. Rekombinanter Antikörper oder Schwer/Leichtkettenkomponente davon, umfassend:
eine schwere Kette umfassend ein C_{H1}-Fragment der konstanten Region, das sich zwischen einer ersten und einer zweiten variablen Domäne befindet, und
eine dritte variable Domäne, die direkt oder indirekt mit der ersten oder zweiten variablen Domäne verknüpft ist,
mit der Maßgabe, dass die schwere Kette nur ein C_{H1} enthält und nur drei variable Domänen enthält, und
eine leichte Kette umfassend mindestens eine C_{L}-Domäne, die sich zwischen einer ersten und einer zweiten variablen Domäne befindet, und eine dritte variable Domäne, die direkt oder indirekt mit der ersten oder zweiten variablen Domäne verknüpft ist,
mit der Maßgabe, dass die leichte Kette nur eine C_{L}-Domäne enthält und nur drei variable Domänen enthält, und
wobei die schwere Kette und die leichte Kette so zueinander ausgerichtet sind, dass sie eine erste Bindungsstelle, die von den ersten variablen Domänen in der leichten und der schweren Kette gebildet wird, eine zweite Bindungsstelle, die von den zweiten variablen Domänen in der leichten und der schweren Kette gebildet wird und eine dritte Bindungsstelle, die von den dritten variablen Domänen in der leichten und der schweren Kette gebildet wird, bereitstellen.

2. Rekombinanter Antikörper oder Schwer/Leichtkettenkomponente davon nach Anspruch 1, wobei C_{H1} mit der ersten variablen Domäne in der schweren Kette fusioniert ist.

3. Rekombinanter Antikörper oder Schwer/Leichtkettenkomponente davon nach Anspruch 1 oder 2, wobei C_{L} mit der ersten variablen Domäne in der leichten Kette fusioniert ist.

4. Rekombinanter Antikörper oder Schwer/Leichtkettenkomponente davon nach einem der Ansprüche 1 bis 3, wobei C_{H1} indirekt mit der zweiten variablen Domäne in der schweren Kette verknüpft ist.

5. Rekombinanter Antikörper oder Schwer/Leichtkettenkomponente davon nach einem der Ansprüche 1 bis 4, wobei C_{L} indirekt mit der zweiten variablen Domäne in der leichten Kette verknüpft ist.

6. Rekombinanter Antikörper oder Schwer/Leichtkettenkomponente davon nach Anspruch 4 oder 5, wobei es sich bei der indirekten Verknüpfung um einen Peptidlinker handelt.

7. Rekombinanter Antikörper oder Schwer/Leichtkettenkomponente davon nach einem der Ansprüche 1 bis 6, wobei die dritte variable Domäne indirekt verknüpft ist.

8. Rekombinanter Antikörper oder Schwer/Leichtkettenkomponente davon nach Anspruch 7, wobei es sich bei der indirekten Verknüpfung um einen Peptidlinker handelt.

9. Rekombinanter Antikörper oder Schwer/Leichtkettenkomponente davon nach einem der Ansprüche 1 bis 8, wobei die dritte variable Domäne in der schweren und/oder der leichten Kette mit der ersten variablen Domäne darin verknüpft ist.

10. Rekombinanter Antikörper oder Schwer/Leichtkettenkomponente davon nach einem der Ansprüche 1 bis 8, wobei die dritte variable Domäne in der schweren und/oder der leichten Kette mit der zweiten variablen Domäne darin verknüpft ist.

11. Rekombinanter Antikörper oder Schwer/Leichtkettenkomponente davon nach einem der Ansprüche 1 bis 10, wobei jede Bindungsstelle von einem VH/VL-Paar der variablen Domäne, bei dem es sich um ein artverwandtes Paar handelt, gebildet wird.

12. Rekombinanter Antikörper oder Schwer/Leichtkettenkomponente davon nach einem der Ansprüche 1 bis 11, wobei jede Bindungsstelle von einem VH/VL-Paar der variablen Domäne gebildet wird, wobei es sich um ein komplementäres VH/VL-Paar, das Antigen kooperativ bindet, handelt.

13. Rekombinanter Antikörper oder Schwer/Leichtkettenkomponente davon nach einem der Ansprüche 1 bis 12, wobei die variablen Domänen von mindestens einer Bindungsstelle über eine Disulfidbrücke verknüpft sind.

14. Rekombinanter Antikörper oder Schwer/Leichtkettenkomponente davon nach einem der Ansprüche 1 bis 13, wobei die zweiten variablen Domänen, die die zweite Bindungsstelle bilden, durch eine Disulfidbrücke stabilisiert sind.

15. Rekombinanter Antikörper oder Schwer/Leichtkettenkomponente davon nach einem der Ansprüche 1 bis 14, wobei dritte variable Domänen, die die dritte Bindungsstelle bilden, durch eine Disulfidbrücke stabilisiert sind.

16. Rekombinanter Antikörper oder Schwer/Leichtkettenkomponente davon nach einem der Ansprüche 1 bis 15, wobei das C_{H1} der schweren Kette mit der C_{L} der leichten Kette über eine Disulfidbrücke verknüpft ist.

17. Rekombinantes Protein nach Anspruch 16, wobei C_{H1} keine Hinge-Region umfasst.

18. Rekombinanter Antikörper oder Schwer/Leichtkettenkomponente davon nach einem der Ansprüche 1 bis 17, die monospezifisch, bispezifisch oder trispezifisch ist.

## Revendications

1. Anticorps recombinant ou composant de chaîne lourde/légère de celui-ci comprenant :
une chaîne lourde comprenant un fragment de région constante C_{H1} situé entre un premier et un deuxième domaine variable, et un troisième domaine variable lié directement ou indirectement au premier ou deuxième domaine variable,
à condition que la chaîne lourde contienne un seul C_{H1} et contient uniquement trois domaines variables, et
une chaîne légère comprenant au moins un domaine C_{L} situé entre un premier et un deuxième domaine variable, et un troisième domaine variable lié directement ou indirectement au premier ou deuxième domaine variable,
à condition que la chaîne légère contienne un seul domaine C_{L} et contienne uniquement trois domaines variables, et
dans lequel lesdites chaînes lourde et légère sont alignées de manière à former un premier site de liaison formé par les premiers domaines variables dans les chaînes légère et lourde, un deuxième site de liaison formé par les deuxièmes domaines variables dans les chaînes légère et lourde, et un troisième site de liaison formé par les troisièmes domaines variables dans les chaînes légère et lourde.

2. Anticorps recombinant ou composant de chaîne lourde/légère de celui-ci selon la revendication 1 dans lequel C_{H1} est fusionné au premier domaine variable dans la chaîne lourde.

3. Anticorps recombinant ou composant de chaîne lourde/légère de celui-ci selon la revendication 1 ou 2 dans lequel C_{L} est fusionné au premier domaine variable dans la chaîne légère.

4. Anticorps recombinant ou composant de chaîne lourde/légère de celui-ci selon l'une quelconque des revendications 1 à 3, dans lequel C_{H1} est lié indirectement au deuxième domaine variable dans la chaîne lourde.

5. Anticorps recombinant ou composant de chaîne lourde/légère de celui-ci selon l'une quelconque des revendications 1 à 4, dans lequel C_{L} est lié indirectement au deuxième domaine variable dans la chaîne légère.

6. Anticorps recombinant ou composant de chaîne lourde/légère de celui-ci selon la revendication 4 ou la revendication 5 dans lequel la liaison indirecte est un lieur peptidique.

7. Anticorps recombinant ou composant de chaîne lourde/légère de celui-ci selon l'une quelconque des revendications 1 à 6, dans lequel le troisième domaine variable est lié indirectement.

8. Anticorps recombinant ou composant de chaîne lourde/légère de celui-ci selon la revendication 7 dans lequel la liaison indirecte est un lieur peptidique.

9. Anticorps recombinant ou composant de chaîne lourde/légère de celui-ci selon l'une quelconque des revendications 1 à 8, dans lequel le troisième domaine variable dans la chaîne lourde et/ou légère est lié au premier domaine variable dans celle-ci.

10. Anticorps recombinant ou composant de chaîne lourde/légère de celui-ci selon l'une quelconque des revendications 1 à 8, dans lequel le troisième domaine variable dans la chaîne lourde et/ou légère est lié au deuxième domaine variable dans celle-ci.

11. Anticorps recombinant ou composant de chaîne lourde/légère de celui-ci selon l'une quelconque des revendications 1 à 10 dans lequel chaque site de liaison est formé par une paire de domaines variables VH/VL qui est une paire apparentée.

12. Anticorps recombinant ou composant de chaîne lourde/légère de celui-ci selon l'une quelconque des revendications 1 à 11 dans lequel chaque site de liaison est formé par une paire de domaines variables VH/VL qui sont une paire VH/VL complémentaire qui se lient en coopération avec un antigène.

13. Anticorps recombinant ou composant de chaîne lourde/légère de celui-ci selon l'une quelconque des revendications 1 à 12, dans lequel les domaines variables d'au moins un site de liaison sont liés par une liaison disulfure.

14. Composant d'anticorps recombinant ou chaîne lourde/légère de celui-ci selon l'une quelconque des revendications 1 à 13 dans lequel les deuxièmes domaines variables formant le deuxième site de liaison sont stabilisés par une liaison disulfure.

15. Anticorps recombinant ou composant de chaîne lourde/légère de celui-ci selon l'une quelconque des revendications 1 à 14, dans lequel les troisièmes domaines variables formant le troisième site de liaison sont stabilisés par une liaison disulfure.

16. Anticorps recombinant ou composant de chaîne lourde/légère de celui-ci selon l'une quelconque des revendications 1 à 15, dans lequel le C_{H1} de la chaîne lourde est lié au C_{L} de la chaîne légère par une liaison disulfure.

17. Protéine recombinante selon la revendication 16, dans laquelle C_{H1} ne comprend pas une région de charnière.

18. Anticorps recombinant ou composant de chaîne lourde/légère de celui-ci selon l'une quelconque des revendications 1 à 17 qui est monospécifique, bispécifique ou trispécifique.


### SEQUENCE LISTING

<110> UCB Pharma SA Adams, Ralph Dave, Emma
<120> Multivalent Antibodies
<130> G0118-WO01
<160> 89
<170> PatentIn version 3.5
<210> 1
   <211> 8
   <212> PRT
   <213> Artificial
<220>
   <223> Hinge Linker
<400> 1
   Asp Lys Thr His Thr Cys Ala Ala
   1               5
<210> 2
   <211> 11
   <212> PRT
   <213> Artificial
<220>
   <223> Hinge Linker
<400> 2
   Asp Lys Thr His Thr Cys Pro Pro Cys Pro Ala
   1               5                   10
<210> 3
   <211> 18
   <212> PRT
   <213> Artificial
<220>
   <223> Hinge Linker
<400> 3
   Asp Lys Thr His Thr Cys Pro Pro Cys Pro Ala Thr Cys Pro Pro Cys
   1               5                   10                  15
   Pro Ala
<210> 4
   <211> 25
   <212> PRT
   <213> Artificial
<220>
   <223> Hinge Linker
<400> 4
   Asp Lys Thr His Thr Cys Pro Pro Cys Pro Ala Thr Cys Pro Pro Cys
   1               5                   10                  15
   Pro Ala Thr Cys Pro Pro Cys Pro Ala
               20                  25
<210> 5
   <211> 30
   <212> PRT
   <213> Artificial
<220>
   <223> Hinge Linker
<400> 5
   Asp Lys Thr His Thr Cys Pro Pro Cys Pro Ala Gly Lys Pro Thr Leu
   1               5                   10                  15
   Tyr Asn Ser Leu Val Met Ser Asp Thr Ala Gly Thr Cys Tyr
               20                  25                  30
<210> 6
   <211> 31
   <212> PRT
   <213> Artificial
<220>
   <223> Hinge Linker
<400> 6
   Asp Lys Thr His Thr Cys Pro Pro Cys Pro Ala Gly Lys Pro Thr His
   1               5                   10                  15
   Val Asn Val Ser Val Val Met Ala Glu Val Asp Gly Thr Cys Tyr
               20                  25                  30
<210> 7
   <211> 15
   <212> PRT
   <213> Artificial
<220>
   <223> Hinge Linker
<400> 7
   Asp Lys Thr His Thr Cys Cys Val Glu Cys Pro Pro Cys Pro Ala
   1               5                   10                  15
<210> 8
   <211> 26
   <212> PRT
   <213> Artificial
<220>
   <223> Hinge Linker
<400> 8
   Asp Lys Thr His Thr Cys Pro Arg Cys Pro Glu Pro Lys Ser Cys Asp
   1               5                   10                  15
   Thr Pro Pro Pro Cys Pro Arg Cys Pro Ala
               20                  25
<210> 9
   <211> 11
   <212> PRT
   <213> Artificial
<220>
   <223> Hinge Linker
<400> 9
   Asp Lys Thr His Thr Cys Pro Ser Cys Pro Ala
   1               5                   10
<210> 10
   <211> 7
   <212> PRT
   <213> Artificial
<220>
   <223> Linker
<400> 10
   Ser Gly Gly Gly Gly Ser Glu
   1               5
<210> 11
   <211> 6
   <212> PRT
   <213> Artificial
<220>
   <223> Linker
<400> 11
   Asp Lys Thr His Thr Ser
   1               5
<210> 12
   <211> 6
   <212> PRT
   <213> Artificial
<220>
   <223> Linker
<400> 12
   Ser Gly Gly Gly Gly Ser
   1               5
<210> 13
   <211> 11
   <212> PRT
   <213> Artificial
<220>
   <223> Linker
<400> 13
   Ser Gly Gly Gly Gly Ser Gly Gly Gly Gly Ser
   1               5                   10
<210> 14
   <211> 16
   <212> PRT
   <213> Artificial
<220>
   <223> Linker
<400> 14
   Ser Gly Gly Gly Gly Ser Gly Gly Gly Gly Ser Gly Gly Gly Gly Ser
   1               5                   10                  15
<210> 15
   <211> 21
   <212> PRT
   <213> Artificial
<220>
   <223> Linker
<400> 15
   Ser Gly Gly Gly Gly Ser Gly Gly Gly Gly Ser Gly Gly Gly Gly Ser
   1               5                   10                  15
   Gly Gly Gly Gly Ser
               20
<210> 16
   <211> 26
   <212> PRT
   <213> Artificial
<220>
   <223> Linker
<400> 16
   Ser Gly Gly Gly Gly Ser Gly Gly Gly Gly Ser Gly Gly Gly Gly Ser
   1               5                   10                  15
   Gly Gly Gly Gly Ser Gly Gly Gly Gly Ser
               20                  25
<210> 17
   <211> 11
   <212> PRT
   <213> Artificial
<220>
   <223> Linker
<400> 17
   Ala Ala Ala Gly Ser Gly Gly Ala Ser Ala Ser
   1               5                   10
<210> 18
   <211> 16
   <212> PRT
   <213> Artificial
<220>
   <223> Linker
<220>
   <221> misc_feature
   <222> (7)..(7)
   <223> Xaa can be any naturally occurring amino acid
<400> 18
   Ala Ala Ala Gly Ser Gly Xaa Gly Gly Gly Ser Gly Ala Ser Ala Ser
   1               5                   10                  15
<210> 19
   <211> 21
   <212> PRT
   <213> Artificial
<220>
   <223> Linker
<220>
   <221> misc_feature
   <222> (7)..(7)
   <223> Xaa can be any naturally occurring amino acid
<220>
   <221> misc_feature
   <222> (12)..(12)
   <223> Xaa can be any naturally occurring amino acid
<400> 19
   Ala Ala Ala Gly Ser Gly Xaa Gly Gly Gly Ser Xaa Gly Gly Gly Ser
   1 5 10 15
   Gly Ala Ser Ala Ser
   20
<210> 20
   <211> 26
   <212> PRT
   <213> Artificial
<220>
   <223> Linker
<220>
   <221> misc_feature
   <222> (7).. (7)
   <223> Xaa can be any naturally occurring amino acid
<220>
   <221> misc_feature
   <222> (12)..(12)
   <223> Xaa can be any naturally occurring amino acid
<220>
   <221> misc_feature
   <222> (17)..(17)
   <223> Xaa can be any naturally occurring amino acid
<400> 20
   Ala Ala Ala Gly Ser Gly Xaa Gly Gly Gly Ser Xaa Gly Gly Gly Ser
   1 5 10 15
   Xaa Gly Gly Gly Ser Gly Ala Ser Ala Ser
   20 25
<210> 21
   <211> 31
   <212> PRT
   <213> Artificial
<220>
   <223> Linker
<220>
   <221> misc_feature
   <222> (7)..(7)
   <223> Xaa can be any naturally occurring amino acid
<220>
   <221> misc_feature
   <222> (12)..(12)
   <223> Xaa can be any naturally occurring amino acid
<220>
   <221> misc_feature
   <222> (17)..(17)
   <223> Xaa can be any naturally occurring amino acid
<220>
   <221> misc_feature
   <222> (22)..(22)
   <223> Xaa can be any naturally occurring amino acid
<400> 21
   Ala Ala Ala Gly Ser Gly Xaa Gly Gly Gly Ser Xaa Gly Gly Gly Ser
   1               5                   10                  15
   Xaa Gly Gly Gly Ser Xaa Gly Gly Gly Ser Gly Ala Ser Ala Ser
               20                  25                  30
<210> 22
   <211> 13
   <212> PRT
   <213> Artificial
<220>
   <223> Linker
<220>
   <221> misc_feature
   <222> (7)..(7)
   <223> Xaa can be any naturally occurring amino acid
<400> 22
   Ala Ala Ala Gly Ser Gly Xaa Ser Gly Ala Ser Ala Ser
   1               5                   10
<210> 23
   <211> 28
   <212> PRT
   <213> Artificial
<220>
   <223> Linker
<400> 23
   Pro Gly Gly Asn Arg Gly Thr Thr Thr Thr Arg Arg Pro Ala Thr Thr
   1 5 10 15
   Thr Gly Ser Ser Pro Gly Pro Thr Gln Ser His Tyr
   20 25
<210> 24
   <211> 11
   <212> PRT
   <213> Artificial
<220>
   <223> Linker
<400> 24
   Ala Thr Thr Thr Gly Ser Ser Pro Gly Pro Thr
   1 5 10
<210> 25
   <211> 6
   <212> PRT
   <213> Artificial
<220>
   <223> Linker
<400> 25
   Ala Thr Thr Thr Gly Ser
   1 5
<210> 26
   <211> 499
   <212> PRT
   <213> Artificial
<220>
   <223> 26gH2gamma1-CH13G4S645gH15G4S1189gH1
<400> 26
<210> 27
   <211> 21
   <212> PRT
   <213> Artificial
<220>
   <223> Linker
<400> 27
   Glu Pro Ser Gly Pro Ile Ser Thr Ile Asn Ser Pro Pro Ser Lys Glu
   1 5 10 15
   Ser His Lys Ser Pro
   20
<210> 28
   <211> 15
   <212> PRT
   <213> Artificial
<220>
   <223> Linker
<400> 28
   Gly Thr Val Ala Ala Pro Ser Val Phe Ile Phe Pro Pro Ser Asp
   1 5 10 15
<210> 29
   <211> 15
   <212> PRT
   <213> Artificial
<220>
   <223> Linker
<400> 29
   Gly Gly Gly Gly Ile Ala Pro Ser Met Val Gly Gly Gly Gly Ser
   1 5 10 15
<210> 30
   <211> 15
   <212> PRT
   <213> Artificial
<220>
   <223> Linker
<400> 30
   Gly Gly Gly Gly Lys Val Glu Gly Ala Gly Gly Gly Gly Gly Ser
   1 5 10 15
<210> 31
   <211> 15
   <212> PRT
   <213> Artificial
<220>
   <223> Linker
<400> 31
   Gly Gly Gly Gly Ser Met Lys Ser His Asp Gly Gly Gly Gly Ser
   1 5 10 15
<210> 32
   <211> 15
   <212> PRT
   <213> Artificial
<220>
   <223> Linker
<400> 32
   Gly Gly Gly Gly Asn Leu Ile Thr Ile Val Gly Gly Gly Gly Ser
   1 5 10 15
<210> 33
   <211> 15
   <212> PRT
   <213> Artificial
<220>
   <223> Linker
<400> 33
   Gly Gly Gly Gly Val Val Pro Ser Leu Pro Gly Gly Gly Gly Ser
   1 5 10 15
<210> 34
   <211> 12
   <212> PRT
   <213> Artificial
<220>
   <223> Linker
<400> 34
   Gly Gly Glu Lys Ser Ile Pro Gly Gly Gly Gly Ser
   1 5 10
<210> 35
   <211> 18
   <212> PRT
   <213> Artificial
<220>
   <223> Linker
<400> 35
   Arg Pro Leu Ser Tyr Arg Pro Pro Phe Pro Phe Gly Phe Pro Ser Val
   1 5 10 15
   Arg Pro
<210> 36
   <211> 18
   <212> PRT
   <213> Artificial
<220>
   <223> Linker
<400> 36
   Tyr Pro Arg Ser Ile Tyr Ile Arg Arg Arg His Pro Ser Pro Ser Leu
   1 5 10 15
   Thr Thr
<210> 37
   <211> 18
   <212> PRT
   <213> Artificial
<220>
   <223> Linker
<400> 37
   Thr Pro Ser His Leu Ser His Ile Leu Pro Ser Phe Gly Leu Pro Thr
   1 5 10 15
   Phe Asn
<210> 38
   <211> 18
   <212> PRT
   <213> Artificial
<220>
   <223> Linker
<400> 38
   Arg Pro Val Ser Pro Phe Thr Phe Pro Arg Leu Ser Asn Ser Trp Leu
   1 5 10 15
   Pro Ala
<210> 39
   <211> 18
   <212> PRT
   <213> Artificial
<220>
   <223> Linker
<400> 39
   Ser Pro Ala Ala His Phe Pro Arg Ser Ile Pro Arg Pro Gly Pro Ile
   1 5 10 15
   Arg Thr
<210> 40
   <211> 18
   <212> PRT
   <213> Artificial
<220>
   <223> Linker
<400> 40
   Ala Pro Gly Pro Ser Ala Pro Ser His Arg Ser Leu Pro Ser Arg Ala
   1 5 10 15
   Phe Gly
<210> 41
   <211> 18
   <212> PRT
   <213> Artificial
<220>
   <223> Linker
<400> 41
   Pro Arg Asn Ser Ile His Phe Leu His Pro Leu Leu Val Ala Pro Leu
   1 5 10 15
   Gly Ala
<210> 42
   <211> 18
   <212> PRT
   <213> Artificial
<220>
   <223> Linker
<400> 42
   Met Pro Ser Leu Ser Gly Val Leu Gln Val Arg Tyr Leu Ser Pro Pro
   1 5 10 15
   Asp Leu
<210> 43
   <211> 18
   <212> PRT
   <213> Artificial
<220>
   <223> Linker
<400> 43
   Ser Pro Gln Tyr Pro Ser Pro Leu Thr Leu Thr Leu Pro Pro His Pro
   1 5 10 15
   Ser Leu
<210> 44
   <211> 18
   <212> PRT
   <213> Artificial
<220>
   <223> Linker
<400> 44
   Asn Pro Ser Leu Asn Pro Pro Ser Tyr Leu His Arg Ala Pro Ser Arg
   1 5 10 15
   Ile Ser
<210> 45
   <211> 17
   <212> PRT
   <213> Artificial
<220>
   <223> Linker
<400> 45
   Leu Pro Trp Arg Thr Ser Leu Leu Pro Ser Leu Pro Leu Arg Arg Arg
   1 5 10 15
   Pro
<210> 46
   <211> 18
   <212> PRT
   <213> Artificial
<220>
   <223> Linker
<400> 46
   Pro Pro Leu Phe Ala Lys Gly Pro Val Gly Leu Leu Ser Arg Ser Phe
   1 5 10 15
   Pro Pro
<210> 47
   <211> 18
   <212> PRT
   <213> Artificial
<220>
   <223> Linker
<400> 47
   Val Pro Pro Ala Pro Val Val Ser Leu Arg Ser Ala His Ala Arg Pro
   1 5 10 15
   Pro Tyr
<210> 48
   <211> 17
   <212> PRT
   <213> Artificial
<220>
   <223> Linker
<400> 48
   Leu Arg Pro Thr Pro Pro Arg Val Arg Ser Tyr Thr Cys Cys Pro Thr
   1 5 10 15
   Pro
<210> 49
   <211> 18
   <212> PRT
   <213> Artificial
<220>
   <223> Linker
<400> 49
   Pro Asn Val Ala His Val Leu Pro Leu Leu Thr Val Pro Trp Asp Asn
   1 5 10 15
   Leu Arg
<210> 50
   <211> 18
   <212> PRT
   <213> Artificial
<220>
   <223> Linker
<400> 50
   Cys Asn Pro Leu Leu Pro Leu Cys Ala Arg Ser Pro Ala Val Arg Thr
   1 5 10 15
   Phe Pro
<210> 51
   <211> 11
   <212> PRT
   <213> Artificial
<220>
   <223> Albumin binding peptide
<400> 51
   Asp Leu Cys Leu Arg Asp Trp Gly Cys Leu Trp
   1 5 10
<210> 52
   <211> 11
   <212> PRT
   <213> Artificial
<220>
   <223> Albumin binding peptide
<400> 52
   Asp Ile Cys Leu Pro Arg Trp Gly Cys Leu Trp
   1               5                   10
<210> 53
   <211> 15
   <212> PRT
   <213> Artificial
<220>
   <223> Albumin binding peptide
<400> 53
   Met Glu Asp Ile Cys Leu Pro Arg Trp Gly Cys Leu Trp Gly Asp
   1               5                   10                  15
<210> 54
   <211> 20
   <212> PRT
   <213> Artificial
<220>
   <223> Albumin binding peptide
<400> 54
   Gln Arg Leu Met Glu Asp Ile Cys Leu Pro Arg Trp Gly Cys Leu Trp
   1               5                   10                  15
   Glu Asp Asp Glu
               20
<210> 55
   <211> 20
   <212> PRT
   <213> Artificial
<220>
   <223> Albumin binding peptide
<400> 55
   Gln Gly Leu Ile Gly Asp Ile Cys Leu Pro Arg Trp Gly Cys Leu Trp
   1               5                   10                  15
   Gly Arg Ser Val
               20
<210> 56
   <211> 21
   <212> PRT
   <213> Artificial
<220>
   <223> Albumin binding peptide
<400> 56
   Gln Gly Leu Ile Gly Asp Ile Cys Leu Pro Arg Trp Gly Cys Leu Trp
   1               5                   10                  15
   Gly Arg Ser Val Lys
               20
<210> 57
   <211> 15
   <212> PRT
   <213> Artificial
<220>
   <223> Albumin binding peptide
<400> 57
   Glu Asp Ile Cys Leu Pro Arg Trp Gly Cys Leu Trp Glu Asp Asp
   1               5                   10                  15
<210> 58
   <211> 18
   <212> PRT
   <213> Artificial
<220>
   <223> Albumin binding peptide
<400> 58
   Arg Leu Met Glu Asp Ile Cys Leu Pro Arg Trp Gly Cys Leu Trp Glu
   1               5                   10                  15
   Asp Asp
<210> 59
   <211> 16
   <212> PRT
   <213> Artificial
<220>
   <223> Albumin binding peptide
<400> 59
   Met Glu Asp Ile Cys Leu Pro Arg Trp Gly Cys Leu Trp Glu Asp Asp
   1 5 10 15
<210> 60
   <211> 15
   <212> PRT
   <213> Artificial
<220>
   <223> Albumin binding peptide
<400> 60
   Met Glu Asp Ile Cys Leu Pro Arg Trp Gly Cys Leu Trp Glu Asp
   1 5 10 15
<210> 61
   <211> 18
   <212> PRT
   <213> Artificial
<220>
   <223> Albumin binding peptide
<400> 61
   Arg Leu Met Glu Asp Ile Cys Leu Ala Arg Trp Gly Cys Leu Trp Glu
   1 5 10 15
   Asp Asp
<210> 62
   <211> 20
   <212> PRT
   <213> Artificial
<220>
   <223> Albumin binding peptide
<400> 62
   Glu Val Arg Ser Phe Cys Thr Arg Trp Pro Ala Glu Lys Ser Cys Lys
   1 5 10 15
   Pro Leu Arg Gly
   20
<210> 63
   <211> 20
   <212> PRT
   <213> Artificial
<220>
   <223> Albumin binding peptide
<400> 63
   Arg Ala Pro Glu Ser Phe Val Cys Tyr Trp Glu Thr Ile Cys Phe Glu
   1 5 10 15
   Arg Ser Glu Gln
   20
<210> 64
   <211> 11
   <212> PRT
   <213> Artificial
<220>
   <223> Albumin binding peptide
<400> 64
   Glu Met Cys Tyr Phe Pro Gly Ile Cys Trp Met
   1 5 10
<210> 65
   <211> 117
   <212> PRT
   <213> Artificial
<220>
   <223> 26 gH2
<400> 65
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Asn Tyr
   20 25 30
   Gly Ile His Trp Ile Arg Gln Ala Pro Gly Lys Gly Leu Glu Trp Val
   35 40 45
   Ala Ser Ile Ser Pro Ser Gly Gly Leu Thr Tyr Tyr Arg Asp Ser Val
   50 55 60
   Lys Gly Arg Phe Thr Ile Ser Arg Asp Asp Ala Lys Asn Ser Pro Tyr
   65 70 75 80
   Leu Gln Met Asn Ser Leu Arg Ala Glu Asp Thr Ala Val Tyr Tyr Cys
   85 90 95
   Ala Thr Gly Gly Glu Gly Ile Phe Asp Tyr Trp Gly Gln Gly Thr Leu
   100 105 110
   Val Thr Val Ser Ser
   115
<210> 66
   <211> 103
   <212> PRT
   <213> Artificial
<220>
   <223> gamma 1 cH1
<400> 66
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val Glu Pro Lys Ser Cys
   100
<210> 67
   <211> 16
   <212> PRT
   <213> Artificial
<220>
   <223> 3G4S
<400> 67
   Ser Gly Gly Gly Gly Ser Gly Gly Gly Gly Ser Gly Gly Gly Gly Ser
   1 5 10 15
<210> 68
   <211> 119
   <212> PRT
   <213> Artificial
<220>
   <223> 645 gH1
<400> 68
   Glu Val Gln Leu Leu Glu Ser Gly Gly Gly Leu Val Gln Pro Gly Gly
   1               5                   10                  15
   Ser Leu Arg Leu Ser Cys Ala Val Ser Gly Ile Asp Leu Ser Asn Tyr
               20                  25                  30
   Ala Ile Asn Trp Val Arg Gln Ala Pro Gly Lys Cys Leu Glu Trp Ile
           35                  40                  45
   Gly Ile Ile Trp Ala Ser Gly Thr Thr Phe Tyr Ala Thr Trp Ala Lys
       50                  55                  60
   Gly Arg Phe Thr Ile Ser Arg Asp Ser Thr Thr Val Tyr Leu Gln Met
   65                  70                  75                  80
   Asn Ser Leu Arg Ala Glu Asp Thr Ala Val Tyr Tyr Cys Ala Arg Thr
                   85                  90                  95
   Val Pro Gly Tyr Ser Thr Ala Pro Tyr Phe Asp Leu Trp Gly Gln Gly
               100                 105                 110
   Thr Leu Val Thr Val Ser Ser
           115
<210> 69
   <211> 25
   <212> PRT
   <213> Artificial
<220>
   <223> 5G4S
<400> 69
   Gly Gly Gly Gly Ser Gly Gly Gly Gly Ser Gly Gly Gly Gly Ser Gly
   1               5                   10                  15
   Gly Gly Gly Ser Gly Gly Gly Gly Ser
               20                  25
<210> 70
   <211> 119
   <212> PRT
   <213> Artificial
<220>
   <223> 1189 gH1
<400> 70
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Asp Tyr
               20                  25                  30
   Asp Met Ala Trp Val Arg Gln Ala Pro Gly Lys Cys Leu Glu Trp Val
           35                  40                  45
   Ala Ser Ile Thr Pro Ser Gly Gly Gly Thr Tyr Tyr Arg Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg His Gly Tyr Thr Leu Asp Tyr Phe Glu Tyr Trp Gly Gln Gly
               100                 105                 110
   Thr Met Val Thr Val Ser Ser
           115
<210> 71
   <211> 5
   <212> PRT
   <213> Artificial
<220>
   <223> 1G4S
<400> 71
   Gly Gly Gly Gly Ser
   1               5
<210> 72
   <211> 10
   <212> PRT
   <213> Artificial
<220>
   <223> 2G4S
<400> 72
   Gly Gly Gly Gly Ser Gly Gly Gly Gly Ser
   1               5                   10
<210> 73
   <211> 15
   <212> PRT
   <213> Artificial
<220>
   <223> 3G4S
<400> 73
   Gly Gly Gly Gly Ser Gly Gly Gly Gly Ser Gly Gly Gly Gly Ser
   1               5                   10                  15
<210> 74
   <211> 20
   <212> PRT
   <213> Artificial
<220>
   <223> 4G4S
<400> 74
   Gly Gly Gly Gly Ser Gly Gly Gly Gly Ser Gly Gly Gly Gly Ser Gly
   1               5                   10                  15
   Gly Gly Gly Ser
               20
<210> 75
   <211> 472
   <212> PRT
   <213> Artificial
<220>
   <223> 26 gL8 CK 3G4S 645gL1 5G4S 1189gL1
<400> 75
   Asp Ile Gln Met Thr Gln Ser Pro Ser Ser Leu Ser Ala Ser Val Gly
   1               5                   10                  15
   Asp Arg Val Thr Ile Thr Cys Arg Ala Thr Gln Ser Ile Tyr Asn Ala
               20                  25                  30
   Leu Ala Trp Tyr Gln Gln Lys Pro Gly Lys Ala Pro Lys Leu Leu Ile
           35                  40                  45
   Tyr Asn Ala Asn Thr Leu His Thr Gly Val Pro Ser Arg Phe Ser Ala
       50                  55                  60
   Ser Gly Ser Gly Thr Asp Ser Thr Leu Thr Ile Ser Ser Leu Gln Pro
   65                  70                  75                  80
   Glu Asp Phe Ala Thr Tyr Tyr Cys Gln Gln Tyr Tyr Asp Tyr Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys Ser Gly Gly Gly Gly Ser Gly Gly Gly Gly
       210                 215                 220
   Ser Gly Gly Gly Gly Ser Asp Ile Val Met Thr Gln Ser Pro Ser Ser
   225                 230                 235                 240
   Val Ser Ala Ser Val Gly Asp Arg Val Thr Ile Thr Cys Gln Ser Ser
   245 250 255
   Pro Ser Val Trp Ser Asn Phe Leu Ser Trp Tyr Gln Gln Lys Pro Gly
   260 265 270
   Lys Ala Pro Lys Leu Leu Ile Tyr Glu Ala Ser Lys Leu Thr Ser Gly
   275 280 285
   Val Pro Ser Arg Phe Lys Gly Ser Gly Ser Gly Thr Asp Phe Thr Leu
   290 295 300
   Thr Ile Ser Ser Leu Gln Pro Glu Asp Phe Ala Thr Tyr Tyr Cys Gly
   305 310 315 320
   Gly Gly Tyr Ser Ser Ile Ser Asp Thr Thr Phe Gly Cys Gly Thr Lys
   325 330 335
   Val Glu Ile Lys Gly Gly Gly Gly Ser Gly Gly Gly Gly Ser Gly Gly
   340 345 350
   Gly Gly Ser Gly Gly Gly Gly Ser Gly Gly Gly Gly Ser Asp Ile Gln
   355 360 365
   Met Thr Gln Ser Pro Ser Ser Leu Ser Ala Ser Val Gly Asp Arg Val
   370 375 380
   Thr Ile Thr Cys Leu Ala Ser Glu Gly Ile Ser Asn Asp Leu Ala Trp
   385 390 395 400
   Tyr Gln Gln Lys Pro Gly Lys Ala Pro Lys Leu Leu Ile Tyr Asp Ala
   405 410 415
   Thr Arg Leu Gln Asp Gly Val Pro Ser Arg Phe Ser Gly Ser Gly Ser
   420 425 430
   Gly Thr Asp Phe Thr Leu Thr Ile Ser Ser Leu Gln Pro Glu Asp Phe
   435 440 445
   Ala Thr Tyr Tyr Cys Gln Gln Ser Tyr Lys Tyr Pro Trp Thr Phe Gly
   450 455 460
   Cys Gly Thr Lys Leu Glu Ile Lys
   465 470
<210> 76
   <211> 107
   <212> PRT
   <213> Artificial
<220>
   <223> 26 gL8
<400> 76
   Asp Ile Gln Met Thr Gln Ser Pro Ser Ser Leu Ser Ala Ser Val Gly
   1               5                   10                  15
   Asp Arg Val Thr Ile Thr Cys Arg Ala Thr Gln Ser Ile Tyr Asn Ala
               20                  25                  30
   Leu Ala Trp Tyr Gln Gln Lys Pro Gly Lys Ala Pro Lys Leu Leu Ile
           35                  40                  45
   Tyr Asn Ala Asn Thr Leu His Thr Gly Val Pro Ser Arg Phe Ser Ala
       50                  55                  60
   Ser Gly Ser Gly Thr Asp Ser Thr Leu Thr Ile Ser Ser Leu Gln Pro
   65                  70                  75                  80
   Glu Asp Phe Ala Thr Tyr Tyr Cys Gln Gln Tyr Tyr Asp Tyr Pro Leu
                   85                  90                  95
   Thr Phe Gly Gly Gly Thr Lys Val Glu Ile Lys
               100                 105
<210> 77
   <211> 107
   <212> PRT
   <213> Artificial
<220>
   <223> CKappa
<400> 77
<210> 78
   <211> 111
   <212> PRT
   <213> Artificial
<220>
   <223> 645 gL1
<400> 78
   Asp Ile Val Met Thr Gln Ser Pro Ser Ser Val Ser Ala Ser Val Gly
   1               5                   10                  15
   Asp Arg Val Thr Ile Thr Cys Gln Ser Ser Pro Ser Val Trp Ser Asn
               20                  25                  30
   Phe Leu Ser Trp Tyr Gln Gln Lys Pro Gly Lys Ala Pro Lys Leu Leu
           35                  40                  45
   Ile Tyr Glu Ala Ser Lys Leu Thr Ser Gly Val Pro Ser Arg Phe Lys
       50                  55                  60
   Gly Ser Gly Ser Gly Thr Asp Phe Thr Leu Thr Ile Ser Ser Leu Gln
   65                  70                  75                  80
   Pro Glu Asp Phe Ala Thr Tyr Tyr Cys Gly Gly Gly Tyr Ser Ser Ile
                   85                  90                  95
   Ser Asp Thr Thr Phe Gly Cys Gly Thr Lys Val Glu Ile Lys Arg
               100                 105                 110
<210> 79
   <211> 107
   <212> PRT
   <213> Artificial
<220>
   <223> 1189 gL1
<400> 79
   Asp Ile Gln Met Thr Gln Ser Pro Ser Ser Leu Ser Ala Ser Val Gly
   1               5                   10                  15
   Asp Arg Val Thr Ile Thr Cys Leu Ala Ser Glu Gly Ile Ser Asn Asp
               20                  25                  30
   Leu Ala Trp Tyr Gln Gln Lys Pro Gly Lys Ala Pro Lys Leu Leu Ile
           35                  40                  45
   Tyr Asp Ala Thr Arg Leu Gln Asp Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Lys Tyr Pro Trp
                   85                  90                  95
   Thr Phe Gly Cys Gly Thr Lys Leu Glu Ile Lys
               100                 105
<210> 80
   <211> 490
   <212> PRT
   <213> Artificial
<220>
   <223> 26Fab gH2 CH1-(3G4S)-645gH1ds-(3xG4S)-652gH2ds
<400> 80
<210> 81
   <211> 463
   <212> PRT
   <213> Artificial
<220>
   <223> 26gL8 CK-(3G4S)-645gL1ds-(3xG4S)-652gL1ds
<400> 81
   Asp Ile Gln Met Thr Gln Ser Pro Ser Ser Leu Ser Ala Ser Val Gly
   1 5 10 15
   Asp Arg Val Thr Ile Thr Cys Arg Ala Thr Gln Ser Ile Tyr Asn Ala
   20 25 30
   Leu Ala Trp Tyr Gln Gln Lys Pro Gly Lys Ala Pro Lys Leu Leu Ile
   35 40 45
   Tyr Asn Ala Asn Thr Leu His Thr Gly Val Pro Ser Arg Phe Ser Ala
   50 55 60
   Ser Gly Ser Gly Thr Asp Ser Thr Leu Thr Ile Ser Ser Leu Gln Pro
   65 70 75 80
   Glu Asp Phe Ala Thr Tyr Tyr Cys Gln Gln Tyr Tyr Asp Tyr Pro Leu
   85 90 95
   Thr Phe Gly Gly Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys Ser Gly Gly Gly Gly Ser Gly Gly Gly Gly
       210                 215                 220
   Ser Gly Gly Gly Gly Ser Asp Ile Val Met Thr Gln Ser Pro Ser Ser
   225                 230                 235                 240
   Val Ser Ala Ser Val Gly Asp Arg Val Thr Ile Thr Cys Gln Ser Ser
                   245                 250                 255
   Pro Ser Val Trp Ser Asn Phe Leu Ser Trp Tyr Gln Gln Lys Pro Gly
               260                 265                 270
   Lys Ala Pro Lys Leu Leu Ile Tyr Glu Ala Ser Lys Leu Thr Ser Gly
           275                 280                 285
   Val Pro Ser Arg Phe Lys Gly Ser Gly Ser Gly Thr Asp Phe Thr Leu
       290                 295                 300
   Thr Ile Ser Ser Leu Gln Pro Glu Asp Phe Ala Thr Tyr Tyr Cys Gly
   305                 310                 315                 320
   Gly Gly Tyr Ser Ser Ile Ser Asp Thr Thr Phe Gly Cys Gly Thr Lys
                   325                 330                 335
   Val Glu Ile Lys Gly Gly Gly Gly Ser Gly Gly Gly Gly Ser Gly Gly
               340                 345                 350
   Gly Gly Ser Asp Ile Gln Met Thr Gln Ser Pro Ser Ser Leu Ser Ala
           355                 360                 365
   Ser Val Gly Asp Arg Val Thr Ile Thr Cys Leu Ala Ser Glu Asp Ile
       370                 375                 380
   Ser Asn Tyr Leu Ala Trp Tyr Gln Gln Lys Pro Gly Lys Ala Pro Lys
   385                 390                 395                 400
   Leu Leu Ile Tyr His Thr Ser Arg Leu Gln Asp Gly Val Pro Ser Arg
   405 410 415
   Phe Ser Gly Ser Gly Ser Gly Thr Asp Phe Thr Leu Thr Ile Ser Ser
   420 425 430
   Leu Gln Pro Glu Asp Phe Ala Thr Tyr Tyr Cys Gln Gln Gly Tyr Arg
   435 440 445
   Phe Pro Leu Thr Phe Gly Cys Gly Thr Lys Val Glu Ile Lys Arg
   450 455 460
<210> 82
   <211> 490
   <212> PRT
   <213> Artificial
<220>
   <223> 26gH2 CH1-(3G4S)-645gH1ds-(3xG4S)-652gH2
<400> 82
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Asn Tyr
   20 25 30
   Gly Ile His Trp Ile Arg Gln Ala Pro Gly Lys Gly Leu Glu Trp Val
   35 40 45
   Ala Ser Ile Ser Pro Ser Gly Gly Leu Thr Tyr Tyr Arg Asp Ser Val
   50 55 60
   Lys Gly Arg Phe Thr Ile Ser Arg Asp Asp Ala Lys Asn Ser Pro Tyr
   65 70 75 80
   Leu Gln Met Asn Ser Leu Arg Ala Glu Asp Thr Ala Val Tyr Tyr Cys
   85 90 95
   Ala Thr Gly Gly Glu Gly Ile Phe Asp Tyr Trp Gly Gln Gly Thr Leu
   100 105 110
   Val Thr Val Ser Ser Ala Ser Thr Lys Gly Pro Ser Val Phe Pro Leu
   115 120 125
   Ala Pro Ser Ser Lys Ser Thr Ser Gly Gly Thr Ala Ala Leu Gly Cys
   130 135 140
   Leu Val Lys Asp Tyr Phe Pro Glu Pro Val Thr Val Ser Trp Asn Ser
   145 150 155 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Lys Val Glu Pro Lys Ser Cys Ser Gly Gly Gly
   210 215 220
   Gly Ser Gly Gly Gly Gly Ser Gly Gly Gly Gly Ser Glu Val Gln Leu
   225 230 235 240
   Leu Glu Ser Gly Gly Gly Leu Val Gln Pro Gly Gly Ser Leu Arg Leu
   245 250 255
   Ser Cys Ala Val Ser Gly Ile Asp Leu Ser Asn Tyr Ala Ile Asn Trp
   260 265 270
   Val Arg Gln Ala Pro Gly Lys Cys Leu Glu Trp Ile Gly Ile Ile Trp
   275 280 285
   Ala Ser Gly Thr Thr Phe Tyr Ala Thr Trp Ala Lys Gly Arg Phe Thr
   290 295 300
   Ile Ser Arg Asp Ser Thr Thr Val Tyr Leu Gln Met Asn Ser Leu Arg
   305 310 315 320
   Ala Glu Asp Thr Ala Val Tyr Tyr Cys Ala Arg Thr Val Pro Gly Tyr
   325 330 335
   Ser Thr Ala Pro Tyr Phe Asp Leu Trp Gly Gln Gly Thr Leu Val Thr
   340 345 350
   Val Ser Ser Gly Gly Gly Gly Ser Gly Gly Gly Gly Ser Gly Gly Gly
   355 360 365
   Gly Ser Gln Val Thr Leu Lys Glu Ser Gly Pro Val Leu Val Lys Pro
   370 375 380
   Thr Glu Thr Leu Thr Leu Thr Cys Thr Val Ser Gly Phe Ser Leu Thr
   385 390 395 400
   Asn Tyr His Val Gln Trp Ile Arg Gln Pro Pro Gly Lys Ala Leu Glu
   405 410 415
   Trp Leu Gly Val Met Trp Ser Asp Gly Asp Thr Ser Phe Asn Ser Val
   420 425 430
   Leu Lys Ser Arg Leu Thr Ile Ser Arg Asp Thr Ser Lys Ser Gln Val
   435 440 445
   Val Leu Thr Met Thr Asn Met Asp Pro Val Asp Thr Ala Thr Tyr Tyr
   450 455 460
   Cys Ala Arg Asp Gly Thr Ile Ala Ala Met Asp Tyr Phe Asp Tyr Trp
   465 470 475 480
   Gly Gln Gly Thr Leu Val Thr Val Ser Ser
   485 490
<210> 83
   <211> 462
   <212> PRT
   <213> Artificial
<220>
   <223> 26gL8 CK-(3G4S)-645gL1ds-(3xG4S)-652gL1
<400> 83
   Asp Ile Gln Met Thr Gln Ser Pro Ser Ser Leu Ser Ala Ser Val Gly
   1               5                   10                  15
   Asp Arg Val Thr Ile Thr Cys Arg Ala Thr Gln Ser Ile Tyr Asn Ala
               20                  25                  30
   Leu Ala Trp Tyr Gln Gln Lys Pro Gly Lys Ala Pro Lys Leu Leu Ile
           35                  40                  45
   Tyr Asn Ala Asn Thr Leu His Thr Gly Val Pro Ser Arg Phe Ser Ala
       50                  55                  60
   Ser Gly Ser Gly Thr Asp Ser Thr Leu Thr Ile Ser Ser Leu Gln Pro
   65                  70                  75                  80
   Glu Asp Phe Ala Thr Tyr Tyr Cys Gln Gln Tyr Tyr Asp Tyr Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Ser Gly Gly Gly Gly Ser Gly Gly Gly Gly
   210 215 220
   Ser Gly Gly Gly Gly Ser Asp Ile Val Met Thr Gln Ser Pro Ser Ser
   225 230 235 240
   Val Ser Ala Ser Val Gly Asp Arg Val Thr Ile Thr Cys Gln Ser Ser
   245 250 255
   Pro Ser Val Trp Ser Asn Phe Leu Ser Trp Tyr Gln Gln Lys Pro Gly
   260 265 270
   Lys Ala Pro Lys Leu Leu Ile Tyr Glu Ala Ser Lys Leu Thr Ser Gly
   275 280 285
   Val Pro Ser Arg Phe Lys Gly Ser Gly Ser Gly Thr Asp Phe Thr Leu
   290 295 300
   Thr Ile Ser Ser Leu Gln Pro Glu Asp Phe Ala Thr Tyr Tyr Cys Gly
   305 310 315 320
   Gly Gly Tyr Ser Ser Ile Ser Asp Thr Thr Phe Gly Cys Gly Thr Lys
   325 330 335
   Val Glu Ile Lys Gly Gly Gly Gly Ser Gly Gly Gly Gly Ser Gly Gly
   340 345 350
   Gly Gly Ser Asp Ile Gln Met Thr Gln Ser Pro Ser Ser Leu Ser Ala
   355 360 365
   Ser Val Gly Asp Arg Val Thr Ile Thr Cys Leu Ala Ser Glu Asp Ile
   370 375 380
   Ser Asn Tyr Leu Ala Trp Tyr Gln Gln Lys Pro Gly Lys Ala Pro Lys
   385 390 395 400
   Leu Leu Ile Tyr His Thr Ser Arg Leu Gln Asp Gly Val Pro Ser Arg
   405 410 415
   Phe Ser Gly Ser Gly Ser Gly Thr Asp Phe Thr Leu Thr Ile Ser Ser
   420 425 430
   Leu Gln Pro Glu Asp Phe Ala Thr Tyr Tyr Cys Gln Gln Gly Tyr Arg
   435 440 445
   Phe Pro Leu Thr Phe Gly Gly Gly Thr Lys Val Glu Ile Lys
   450 455 460
<210> 84
   <211> 488
   <212> PRT
   <213> Artificial
<220>
   <223> 26gH2 CH1-(3G4S)-645gH1ds-(3xG4S)-870ds
<400> 84
<210> 85
   <211> 463
   <212> PRT
   <213> Artificial
<220>
   <223> 26gL8 CK-(3G4S)-645gL1ds-(3xG4S)-870ds
<400> 85
   Asp Ile Gln Met Thr Gln Ser Pro Ser Ser Leu Ser Ala Ser Val Gly
   1               5                   10                  15
   Asp Arg Val Thr Ile Thr Cys Arg Ala Thr Gln Ser Ile Tyr Asn Ala
               20                  25                  30
   Leu Ala Trp Tyr Gln Gln Lys Pro Gly Lys Ala Pro Lys Leu Leu Ile
           35                  40                  45
   Tyr Asn Ala Asn Thr Leu His Thr Gly Val Pro Ser Arg Phe Ser Ala
       50                  55                  60
   Ser Gly Ser Gly Thr Asp Ser Thr Leu Thr Ile Ser Ser Leu Gln Pro
   65                  70                  75                      80
   Glu Asp Phe Ala Thr Tyr Tyr Cys Gln Gln Tyr Tyr Asp Tyr Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Ser Gly Gly Gly Gly Ser Gly Gly Gly Gly
   210 215 220
   Ser Gly Gly Gly Gly Ser Asp Ile Val Met Thr Gln Ser Pro Ser Ser
   225 230 235 240
   Val Ser Ala Ser Val Gly Asp Arg Val Thr Ile Thr Cys Gln Ser Ser
   245 250 255
   Pro Ser Val Trp Ser Asn Phe Leu Ser Trp Tyr Gln Gln Lys Pro Gly
   260 265 270
   Lys Ala Pro Lys Leu Leu Ile Tyr Glu Ala Ser Lys Leu Thr Ser Gly
   275 280 285
   Val Pro Ser Arg Phe Lys Gly Ser Gly Ser Gly Thr Asp Phe Thr Leu
   290 295 300
   Thr Ile Ser Ser Leu Gln Pro Glu Asp Phe Ala Thr Tyr Tyr Cys Gly
   305 310 315 320
   Gly Gly Tyr Ser Ser Ile Ser Asp Thr Thr Phe Gly Cys Gly Thr Lys
   325 330 335
   Val Glu Ile Lys Gly Gly Gly Gly Ser Gly Gly Gly Gly Ser Gly Gly
   340 345 350
   Gly Gly Ser Asp Ile Gln Met Thr Gln Ser Pro Ser Ser Leu Ser Ala
   355 360 365
   Ser Val Gly Asp Arg Val Thr Ile Thr Cys Lys Ala Ser Gln Asn Val
   370 375 380
   Gly Thr Asn Val Ala Trp Tyr Gln Gln Lys Pro Gly Lys Ala Pro Lys
   385 390 395 400
   Ala Leu Ile Tyr Ser Ala Ser Phe Leu Tyr Ser Gly Val Pro Tyr Arg
   405 410 415
   Phe Ser Gly Ser Gly Ser Gly Thr Asp Phe Thr Leu Thr Ile Ser Ser
   420 425 430
   Leu Gln Pro Glu Asp Phe Ala Thr Tyr Tyr Cys Gln Gln Tyr Asn Ile
   435 440 445
   Tyr Pro Leu Thr Phe Gly Cys Gly Thr Lys Val Glu Ile Lys Arg
   450 455 460
<210> 86
   <211> 488
   <212> PRT
   <213> Artificial
<220>
   <223> 26gH2 CH1-(3G4S)-645gH1ds-(3xG4S)-870
<400> 86
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Thr Asn Tyr
               20                  25                  30
   Gly Ile His Trp Ile Arg Gln Ala Pro Gly Lys Gly Leu Glu Trp Val
           35                  40                  45
   Ala Ser Ile Ser Pro Ser Gly Gly Leu Thr Tyr Tyr Arg Asp Ser Val
       50                  55                  60
   Lys Gly Arg Phe Thr Ile Ser Arg Asp Asp Ala Lys Asn Ser Pro Tyr
   65                  70                  75                  80
   Leu Gln Met Asn Ser Leu Arg Ala Glu Asp Thr Ala Val Tyr Tyr Cys
                   85                  90                  95
   Ala Thr Gly Gly Glu Gly Ile Phe Asp Tyr Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
   165 170 175
   Ser Gly Leu Tyr Ser Leu Ser Ser Val Val Thr Val Pro Ser Ser Ser
   180 185 190
   Leu Gly Thr Gln Thr Tyr Ile Cys Asn Val Asn His Lys Pro Ser Asn
   195 200 205
   Thr Lys Val Asp Lys Lys Val Glu Pro Lys Ser Cys Ser Gly Gly Gly
   210 215 220
   Gly Ser Gly Gly Gly Gly Ser Gly Gly Gly Gly Ser Glu Val Gln Leu
   225 230 235 240
   Leu Glu Ser Gly Gly Gly Leu Val Gln Pro Gly Gly Ser Leu Arg Leu
   245 250 255
   Ser Cys Ala Val Ser Gly Ile Asp Leu Ser Asn Tyr Ala Ile Asn Trp
   260 265 270
   Val Arg Gln Ala Pro Gly Lys Cys Leu Glu Trp Ile Gly Ile Ile Trp
   275 280 285
   Ala Ser Gly Thr Thr Phe Tyr Ala Thr Trp Ala Lys Gly Arg Phe Thr
   290 295 300
   Ile Ser Arg Asp Ser Thr Thr Val Tyr Leu Gln Met Asn Ser Leu Arg
   305 310 315 320
   Ala Glu Asp Thr Ala Val Tyr Tyr Cys Ala Arg Thr Val Pro Gly Tyr
   325 330 335
   Ser Thr Ala Pro Tyr Phe Asp Leu Trp Gly Gln Gly Thr Leu Val Thr
   340 345 350
   Val Ser Ser Gly Gly Gly Gly Ser Gly Gly Gly Gly Ser Gly Gly Gly
   355 360 365
   Gly Ser Glu Val Gln Leu Val Glu Ser Gly Gly Gly Leu Val Gln Pro
   370 375 380
   Gly Gly Ser Leu Arg Leu Ser Cys Ala Ala Ser Gly Tyr Val Phe Thr
   385 390 395 400
   Asp Tyr Gly Met Asn Trp Val Arg Gln Ala Pro Gly Lys Gly Leu Glu
   405 410 415
   Trp Met Gly Trp Ile Asn Thr Tyr Ile Gly Glu Pro Ile Tyr Ala Asp
               420                 425                 430
   Ser Val Lys Gly Arg Phe Thr Phe Ser Leu Asp Thr Ser Lys Ser Thr
           435                 440                 445
   Ala Tyr Leu Gln Met Asn Ser Leu Arg Ala Glu Asp Thr Ala Val Tyr
       450                 455                 460
   Tyr Cys Ala Arg Gly Tyr Arg Ser Tyr Ala Met Asp Tyr Trp Gly Gln
   465                 470                 475                 480
   Gly Thr Leu Val Thr Val Ser Ser
                   485
<210> 87
   <211> 462
   <212> PRT
   <213> Artificial
<220>
   <223> 26gL8 CK-(3G4S)-645gL1ds-(3xG4S)-870
<400> 87
   Asp Ile Gln Met Thr Gln Ser Pro Ser Ser Leu Ser Ala Ser Val Gly
   1               5                   10                  15
   Asp Arg Val Thr Ile Thr Cys Arg Ala Thr Gln Ser Ile Tyr Asn Ala
               20                  25                  30
   Leu Ala Trp Tyr Gln Gln Lys Pro Gly Lys Ala Pro Lys Leu Leu Ile
           35                  40                  45
   Tyr Asn Ala Asn Thr Leu His Thr Gly Val Pro Ser Arg Phe Ser Ala
       50                  55                  60
   Ser Gly Ser Gly Thr Asp Ser Thr Leu Thr Ile Ser Ser Leu Gln Pro
   65                  70                  75                  80
   Glu Asp Phe Ala Thr Tyr Tyr Cys Gln Gln Tyr Tyr Asp Tyr Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys Ser Gly Gly Gly Gly Ser Gly Gly Gly Gly
   210 215 220
   Ser Gly Gly Gly Gly Ser Asp Ile Val Met Thr Gln Ser Pro Ser Ser
   225 230 235 240
   Val Ser Ala Ser Val Gly Asp Arg Val Thr Ile Thr Cys Gln Ser Ser
   245 250 255
   Pro Ser Val Trp Ser Asn Phe Leu Ser Trp Tyr Gln Gln Lys Pro Gly
   260 265 270
   Lys Ala Pro Lys Leu Leu Ile Tyr Glu Ala Ser Lys Leu Thr Ser Gly
   275 280 285
   Val Pro Ser Arg Phe Lys Gly Ser Gly Ser Gly Thr Asp Phe Thr Leu
   290 295 300
   Thr Ile Ser Ser Leu Gln Pro Glu Asp Phe Ala Thr Tyr Tyr Cys Gly
   305 310 315 320
   Gly Gly Tyr Ser Ser Ile Ser Asp Thr Thr Phe Gly Cys Gly Thr Lys
   325 330 335
   Val Glu Ile Lys Gly Gly Gly Gly Ser Gly Gly Gly Gly Ser Gly Gly
   340 345 350
   Gly Gly Ser Asp Ile Gln Met Thr Gln Ser Pro Ser Ser Leu Ser Ala
   355 360 365
   Ser Val Gly Asp Arg Val Thr Ile Thr Cys Lys Ala Ser Gln Asn Val
   370 375 380
   Gly Thr Asn Val Ala Trp Tyr Gln Gln Lys Pro Gly Lys Ala Pro Lys
   385 390 395 400
   Ala Leu Ile Tyr Ser Ala Ser Phe Leu Tyr Ser Gly Val Pro Tyr Arg
   405 410 415
   Phe Ser Gly Ser Gly Ser Gly Thr Asp Phe Thr Leu Thr Ile Ser Ser
   420 425 430
   Leu Gln Pro Glu Asp Phe Ala Thr Tyr Tyr Cys Gln Gln Tyr Asn Ile
   435 440 445
   Tyr Pro Leu Thr Phe Gly Gln Gly Thr Lys Val Glu Ile Lys
   450 455 460
<210> 88
   <211> 11
   <212> PRT
   <213> Artificial
<220>
   <223> linker
<400> 88
   Gly Ala Pro Ala Pro Ala Ala Pro Ala Pro Ala
   1 5 10
<210> 89
   <211> 4
   <212> PRT
   <213> Artificial
<220>
   <223> linker
<400> 89
   Pro Pro Pro Pro
   1